(19) Europäisches Patentamt
European Patent Office
Office européen des brevets

(11) Veröffentlichungsnummer : **0 363 659 B1**

(12) # EUROPÄISCHE PATENTSCHRIFT

(45) Veröffentlichungstag der Patentschrift :
**20.01.93 Patentblatt 93/03**

(51) Int. Cl.$^5$ : **C07C 15/52,** C07C 15/58, C07C 43/215, C07C 211/45, C07D 213/16, C08F 4/42

(21) Anmeldenummer : **89116814.8**

(22) Anmeldetag : **12.09.89**

(54) **Verwendung von Stilbenverbindungen bei der anionischen Polymerisation.**

(30) Priorität : **22.09.88 DE 3832204**

(43) Veröffentlichungstag der Anmeldung :
**18.04.90 Patentblatt 90/16**

(45) Bekanntmachung des Hinweises auf die Patenterteilung :
**20.01.93 Patentblatt 93/03**

(84) Benannte Vertragsstaaten :
**BE DE FR GB IT NL**

(56) Entgegenhaltungen :
**DE-A- 2 241 304**
**GB-A- 2 121 789**

(73) Patentinhaber : **BASF Aktiengesellschaft**
**Carl-Bosch-Strasse 38**
**W-6700 Ludwigshafen (DE)**

(72) Erfinder : **Bender, Dietmar, Dr.**
**Sebastian-Kneipp-Strasse 19**
**W-6707 Schifferstadt (DE)**
Erfinder : **Bronstert, Klaus, Dr.**
**Gartenstrasse 26**
**W.6719 Carlsberg (DE)**

EP 0 363 659 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf die Verwendung gewisser konjugierter aromatischer oder quasiaromatischer Verbindungen der allgemeinen Formel I (a, b oder c)

$$R^1R^2R^3Ar^1\text{-}CH{=}CH\text{-}Ar^2R^4R^5R^6 \quad (Ia)$$

$$R^1R^2R^3Ar^1\text{-}CH{=}CH\text{-}Ar^2R^4R^5R^6\text{-}CH{=}CH\text{-}Ar^3R^7R^8R^9 \quad (Ib)$$

$$R^1R^2R^3Ar^1\text{-}CH{=}CH\text{-}Ar^2(CH_2)_nAr^3\text{-}CH{=}CH\text{-}Ar^4R^4R^5R^6 \quad (Ic)$$

wobei $Ar^1$ einen ein- oder mehrbindigen aromatischen oder quasiaromatischen Rest bedeutet, bei der anionischen Polymerisation und deren Herstellung.

Stilbene oder Verbindungen, die als Stilbenderivate aufgefaßt werden können, finden technisches Interesse als Scintillatoren, optische Aufheller (vgl. DE-A-2 241 504; D1), Flüssigkristalle, Photoleiter (vgl. GB-A-2 121 789; D2) und als Initiatoren für die anionische Polymerisation. Für den letztgenannten Einsatzbereich muß die Stammverbindung durch Reduktion mit Alkalimetall in ein carbanionisches Derivat überführt werden, das mit bestimmten olefinisch ungesättigten Monomeren (z.B. Styrol) zu einem sog. living-polymer reagiert.

Die Aufgabe der Erfindung ist die Bereitstellung von Alkalimetallverbindungen von Stilbenen Ia, Ib, die längere Alkylketten oder ähnliche Gruppen als unmittelbare oder mittelbare Substituenten ($R^1$ bis $R^9$ in den Formeln Ia, Ib) aufweisen und daher löslich in Kohlewasserstoffen sind, und von stilbenähnlichen Verbindungen der allgemeinen Formel Ic, die aus Alkylenbisaryl-Verbindungen und z.B. entsprechenden Toluolderivaten entstanden zu denken sind; sie haben die Struktur

$$(R^1R^2R^3Ar^1\text{-}CH{=}CH\text{-}Ar^2(CH_2)_n\text{-}Ar^3\text{-}CH{=}CH\text{-}Ar^4(R^4R^5R^6)$$

wobei n eine Zahl von wenigstens 4 bedeutet, wenn kein anderer Rest $R^1$ bis $R^6$ zugegen ist, der löslichkeitsvermittelnd ist. In den übrigen Fällen kann n Null oder eine ganze Zahl von bis zu 20 sein.

Erfindungsgegenstand ist die Verwendung einer mit Alkalimetall umgesetzten Stilben-Verbindung der allgemeinen Formel Ia, Ib oder Ic, in der $Ar^1$ bis $Ar^4$ gleiche oder verschiedene aromatische oder quasiaromatische Reste und mindestens einer der Reste $R^1$ bis $R^6$ bzw. $R^1$ bis $R^9$ Alkyl, Alkoxy, Dialkyl- oder Diarylamino mit jeweils wenigstens 4 C-Atomen bedeutet und/oder n mindestens den Wert 4 hat, als Katalysator für die anionische Polymerisation. Soweit die vorstehenden Verbindungen (in nicht metallierter Form) bekannt sind, etwa aus D1 oder D2, ist ihre Verwendung nicht erwähnt und die Vorteile ihrer Verwendung als Polymerisationskatalysatoren nicht erkannt worden.

Zur Herstellung der Verbindungen ist im einzelnen das Folgende zu sagen:

Es ist bekannt, daß Verbindungen vom Typus der allgemeinen Formel I durch Carbonylolefinierung nach Wittig (Angew. Makromol. Chem. 29/30, 307, 1973; Chem. Ber. 94, 907, 1961; J. Org. Chem. 24, 1246, 1959) aus einem Aldehyd und einem Phosphoniumylid sowie durch Umsetzung von Grignard-Verbindungen mit Aldehyden (Recueil des Traveaux Chimiques Pays-Bas 72, 765 (1953)) und über den Weg der sog. Anilsynthese (Hel. Chim. Acta, 52 (8), 2521 (1969), Hel. Chim. Acta, 63 (5), 1311, (1980)) zugänglich sind.

Für eine technische Synthese der Zielverbindungen ist die gute verfügbarkeit der Ausgangssubstanzen eine notwendige Voraussetzung, die jedoch bisher weitgehend fehlt:

Im Fall der Wittig-Reaktion und der Umsetzung von Grignard-verbindungen müssen schwer zugängliche, halogenmethylierte Ausgangsstoffe eingesetzt werden. Die Synthese von Alkyl, und Alkoxy-substituierten Chlormethylaromaten erfolgt bevorzugt durch Chlormethylierung, die jedoch aus gewerbehygienischen Gründen praktisch nicht handhabbar ist.

Die sog. Anil-Synthese ermöglicht an sich den Einsatz der entsprechenden Methylaromaten, die durch Friedel-Crafts-Alkylierung gut zugänglich sind. So lassen sich nach dem Anil-Verfahren methylsubstituierte carbo- und heterocyclische Aromaten der Diphenyl-, Terphenyl-, Stilben-, Naphthalin-, Anthracen-, Phenanthren und Pyridin-Reihe in einer Ausbeute von 20 - 80 % mit entsprechenden Benzalanilinen zu Verbindungen des Strukturtyps I umsetzen. Vom einfachen Toluol ausgehend, gelingt die Darstellung von Stilbenen jedoch mit einer Ausbeute von höchstens 15 %. Die Anilsynthese muß deshalb als ein Syntheseverfahren angesehen werden, das vor allem auf konjugierte und kondensierte Systeme sowie heterocyclische Aromaten anwendbar ist.

Die erfindungsgemäßen Verbindungen erhält man vorteilhaft nach einer in J. Org. Chem 35, (1970), 1288 beiläufig erwähnten Methode ausgehend von einfach zugänglichen, Methylgruppen tragenden aromatischen Verbindungen, die sich vom Benzol, Pyridin und höheren konjugierten und kondensierten aromatischen Systemen ableiten lassen und jeweils einem geeigneten Aldehyd. Der im folgenden eingehend beschriebene Verfahrensweg eignet sich mit besonderem Vorteil zur Gewinnung von Stilbenen oder stilbenanalogen Verbindungen, die substituierte Arylreste, z.B. des Benzols, Naphthalins oder Pyridins aufweisen, insbesondere z.B. solche mit längerkettigen Alkylresten oder Dialkyl- oder Diarylaminogruppen als Substituenten, d.h. Verbindungen der allgemeinen Struktur Ib oder Ic.

Das Verfahren zur Herstellung von Verbindungen des Formeltyps Ia und Ib, umfaßt folgende Verfahrensschritte

- man metalliert Toluol, Xylol oder eine analoge, mindestens eine Methylgruppe tragende aromatische oder quasiaromatische Verbindung ein- bzw. zweifach;
- man setzt die metallierte (jeweils ggf. durch $R^1$ bis $R^9$ substituierte) Verbindung $ArCH_2M$ bzw. $Ar(CH_2M)_2$ mit einem entsprechenden (wiederum ggf. durch $R^1$ bis $R^9$ substituierten) aromatischen Aldehyd ArCHO oder Dialdehyd $Ar(CHO)_2$ um, wobei entsprechende Mono- oder Dialkoholate IIa, IIb, IIb′ gebildet werden.

$$R^1R^2R^3ArCH_2CH(OM)ArR^4R^5R^6 \qquad IIa$$

$$R^1R^2R^3ArCH(OM)CH_2ArR^4R^5R^6CH_2CH(OM)ArR^7R^8R^9 \qquad IIb$$

$$R^1R^2R^3ArCH_2CH(OM)ArR^4R^5R^6CH(OM)CH_2ArR^7R^8R^9 \qquad IIb'$$

und

- man hydrolysiert oder solvolysiert IIa, IIb bzw. IIb′ oder führt IIa, IIb bzw. IIb′ in an sich bekannter Weise in eine Esterverbindung über und
- dehydratisiert oder pyrolysiert die erhaltene Zwischenverbindung.

Verbindungen Ic können auf entsprechende Weise erhalten werden.

Schlüsselverbindungen sind die auftretenden Zwischenprodukte, d.h. die metallierten Methylaromaten und die durch Hydrolyse/Solvolyse erhaltenen Hydroxyverbindungen oder Ester-Verbindungen IIa, IIb bzw. IIb′.

Als Arylreste im Sinne der Erfindung kommen beispielsweise in Frage die Reste des Benzols, Pyridins, Naphthalins, Anthracens, Phenanthrens, Chinolins oder Isochinolins, Stilben selbst, Biphenyl, Diphenyl(thio)-ether, Diphenylalkane, Terphenyl, Furan, sowie Paare von gleichen oder verschiedenen Arylresten, die jeweils durch eine längere Alkylenkette getrennt sind.

Der aromatische Grundkörper kann dabei durch eine oder mehrere löslichkeitsvermittelnde Gruppen R substituiert sein, welche sich unter den Bedingungen der Metallierungsreaktion gegenüber den verwendeten metallorganischen Basen chemisch indifferent verhalten. Geeignete Substituenten sind lineare oder verzweigte Alkyl-, Alkenyl-, Aralkyl-, Aryl- oder Cycloalkylreste mit z.B. bis zu 20 Kohlenstoffatomen, die auch Etherbrücken enthalten können, ferner N,N-Dialkylaminoreste mit bis zu 20 C-Atomen je Alkylgruppe. Die Alkylsubstituenten können außerdem über zwei Positionen mit dem aromatischen System verknüpft sein und somit einen alicyclischen Ring bilden. Schließlich können jeweils zwei Arylreste durch einen löslichkeitsvermittelnden Alkylenrest voneinander getrennt sein. Die Metallierung der Substituenten unterbleibt i.a., wenn nicht ein zu hoher Überschuß an Metallierungsmittel angewendet wird.

In Verbindungen der allgemeinen Struktur $Ar\text{-}CH_3$ und $CH_3\text{-}Ar\text{-}CH_3$ bedeutet Ar bevorzugt eine Phenyl-, Naphthyl- oder Pyridingruppe, die durch einen oder zwei Alkyl- oder N,N-Dialkylaminoreste in der Form substituiert sind, daß die Gesamtzahl der Kohlenstoffatome im Substituenten im Bereich von 4 bis zu 60 Kohlenstoffatomen, vorzugsweise 4 bis 30 Kohlenstoffatomen liegt. Die Ausgangsstoffe für die Synthese werden - für die Gewinnung von Initiatoren für die anionische Polymerisation - zweckmäßig so gewählt, daß man Stilbene oder Bis-Stilbene erhält, die längerkettige Substituenten vom Alkantyp bzw. entsprechende Zwischenstücke, also Alkylenbrücken aufweisen (mindestens etwa 6 - 8 C-Atome lang)- die in Kohlenwasserstoffen noch als Mono- oder Poly-Anionen löslich sind.

Die Metallierung der Methylgruppen tragenden Aromaten kann an jeder vorhandenen Methylgruppe verlaufen nach

$$Ar\text{-}CH_3 + MBs \rightarrow Ar\text{-}CH_2M + H\text{-}Bs$$

$$H_3C\text{-}Ar\text{-}CH_3 + 2\ MBs \rightarrow MCH_2\text{-}Ar\text{-}CH_2M + 2\ HBs,$$

wobei Bs den Rest der verwendeten metallorganischen Base und M vorzugsweise ein Alkalimetall, insbesondere Lithium oder Kalium bedeutet.

Geeignete metallorganische Basen sind mehrfach beschrieben worden (Angew. Chem. 85, 544, (1973); J. Am. Chem. Soc. 99, 1473 (1977); J. Org. Chem. 51, 1618, (1986); J. Org. Chem. 47, 3949, (1982); J. Org. Chem. 38, 1491, (1973). Die dort beschriebenen Experimente beschränken sich jedoch auf die Metallierung ausschließlich methylsubstituierter Aromaten, die spektroskopische Charakterisierung der Organometallverbindungen und deren Umsetzung mit einfachen Elektrophilen- wie beispielsweise Dimethylsulfat. Das präparative Potential dieser nucleophilen Verbindungen im Hinblick auf Umsetzungen mit aromatischen Aldehyden wurde nicht untersucht. Ebensowenig wurde die Frage geklärt, inwieweit die Regioselektivität der Metallierungsreaktion durch die Anwesenheit weiterer Substituenten, wie beispielsweise Alkylgruppen beeinflußt wird.

Als metallorganische Base für die Wasserstoffabstraktion eignen sich insbesondere Mischungen aus Kaliumalkoholaten und Lithiumalkylen. Bevorzugt sind Mischungen der Kaliumsalze linearer, verzweigter oder cyclischer Alkylalkoholate mit 1 bis 22 Kohlenstoffatomen und Lithiumalkylen wie z.B. Ethyllithium n- oder sec.-Butyllithium. Besonders geeignet sind Mischungen aus n- oder sec.-Butyllithium und Kalium-tert.-butanolat.

Es wird angenommen, daß die Metallierungsreaktion über die Zwischenstufe einer Organokalium-verbindung führt.

Das Mischungsverhältnis von Kaliumalkoholat zu Lithiumalkyl kann zwar in weiten Grenzen variiert werden, da jedoch nur der gemischte 1:1-Komplex als Metallierungsagens wirkt, ist es sinnvoll, stöchiometische Mengen an Kaliumalkoholat und Lithiumalkylen zu verwenden.

Die Menge der Kaliumalkoholat/Lithiumalkyl-Base kann, bezogen auf die Methylgruppen tragende Komponente, im Bereich von 0-1 - 1,5 mol je mol Methylgruppe variiert werden. Um einen quantitativen Umsatz aller Methylgruppen zu gewährleisten, verwendet man die Kaliumalkoholat/Lithiumalkyl-Base zweckmäßig in einer Menge von 1 - 1,5 mol je mol Methylgruppe.

Als Lösungsmittel für die Metallierungsreaktion eignen sich geradkettige und verzweigte aliphatische Kohlenwasserstoffen, wie z.B. n-Octan oder n-Hexan sowie einfache oder substituierte cycloaliphatische Kohlenwasserstoffe, wie z.B. Cyclohexan oder Methylcyclohexan sowie jede beliebige Mischung der aliphatischen und cycloaliphatischen Kohlenwasserstoffe. Aromatische Kohlenwasserstoffe können logischerweise dann verwendet werden, wenn sie nicht durch Methylgruppen kernsubstituiert sind. Die Umsetzung verläuft schon bei einer Temperatur von -20°C an, bis zu 120°C, bevorzugt ist der Temperaturbereich von 20 - 80°C.

Die Geschwindigkeit der Metallierungsreaktion wird entscheidend durch die verwendete Kaliumalkoholat- und Lithiumalkyl-Komponente, die Methylgruppen tragende Komponente sowie die Reaktionstemperatur bestimmt.

Beispielsweise erfolgt die vollständige Umsetzung von Decyl- oder Hexadecyltoluol in Cyclohexan mit einer Mischung aus Kalium-tert.-butanolat/n-Butyllithium bei 40°C binnen 60 Minuten. Es entsteht eine intensiv rot gefärbte Lösung. Kurzkettig alkylsubstituierte Toluole fallen dagegen nach der Metallierung in aliphatischen Kohlenwasserstoffen aus und bilden rote Suspensionen oder hochviskose- braunschwarze Abscheidungen.

Die Dimetallierung entsprechender Dimethylarylene erfordert eine vergleichsweise längere Reaktionszeit bzw. höhere Reaktionstemperatur. Im Fall des Hexadecyl-meta-xylols gelingt die doppelte Wasserstoffabstraktion mit einer Kaliumalkoholat/Lithiumalkyl-Base nach 4-stündigem Erwärmen auf 70°C. Die entstehende Dikaliumverbindung ist im Gegensatz zur Monokaliumverbindung des Hexadecyl-toluols in Cyclohexan unlöslich und bildet eine dunkelrot/braune Suspension.

Für die Metallierung der Methylgruppen tragenden Komponente mit Lithiumalkylen in Anwesenheit sog. mehrzähniger Komplexierungsmittel, wie Kronenether oder N,N,N',N'-Tetramethylethylendiamin eignet sich eine Mischung, die je mol Methylengruppen 0,05 - 1,5 mol Lithiumalkyl und 0,05 - 10 mol des Komplexierungsmittel enthält. Bevorzugt werden je Mol Methylgruppen, 1 - 1,2 mol n- oder sec.-Butyllithium und 1 - 3 mol N,N,N'-N'-Tetramethylethylendiamin eingesetzt.

Die Reaktionstemperatur liegt wiederum zwischen 0 - 100°C, vorzugsweise im Bereich von 20 - 70°C.

Die Geschwindigkeit der Wasserstoffabstraktion wird ebenfalls von der Methylgruppen tragende Komponente und der Reaktionstemperatur bestimmt, i.a. wird eine Reaktionszeit von 1 - 8 Stunden benötigt.

Zur Herstellung von Verbindungen mit der Formel I (a, b oder c) wird die erhaltene metallorganische Verbindung mit einem aromatischen Aldehyd umgesetzt:

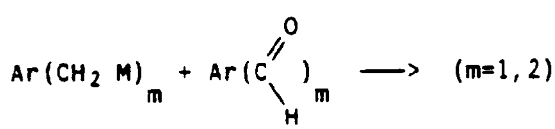


Man erhält ein Alkoholat der Formel IIa bzw. IIb bzw. IIc

$ArCH_2CH(OM)Ar$ IIa

$ArCH(OM)CH_2ArCH_2CH(OM)Ar$ IIb

$ArCH_2CH(OM)ArCH(OM)CH_2Ar$ IIb'

dessen Alkoholatfunktion - am besten in situ - durch Umsetzung mit Essigsäureanhydrid, Wasser, Acetylsäurechlorid- Schwefelkohlenstoff/Methyljodid oder Chlorkohlensäureestern in Hydroxyl oder eine thermisch oder katalytisch elimierbare Gruppe der allgemeinen Formel -OE umgewandelt wird. Bevorzugt werden Wasser oder Essigsäureanhydrid, wobei im Fall des Essigsäureanhydrids Mit einem 1 - 2fachen Überschuß gearbeitet wird.

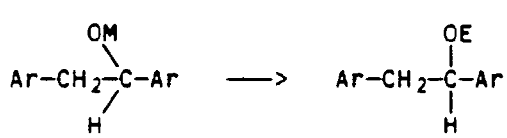

Dabei bedeutet E Wasserstoff oder eine eliminierbare Gruppe, z.B. einen Acyl- (insbesondere Acetyl-) oder

(Thio)kohlensäurerest. Bei der Umsetzung der metallorganischen Verbindung mit dem Aldehyd werden zweckmäßig stöchiometrische Mengen der Reaktionspartner eingesetzt. Es ist aber auch möglich, einen Überschuß eines Partners einzusetzen. Der Aldehyd wird unverdünnt oder als konzentrierte Lösung in einem Lösungsmittel, wie beispielsweise Diethylether oder Benzol, zudosiert. Die Umsetzung läuft schon bei einer Temperatur im Bereich von -30 bis -40°C ab. Nach der Zugabe des Aldehyds verliert die Suspension/Lösung der metallorganischen Verbindung ihre intensive Farbe.

Nach der Aufarbeitung wird die Zwischenverbindung auf katalytischem oder thermischem Wege (Houben-Weyl Bd. 5/1b) in die erfindungsgemäße Zielverbindung der allgemeinen Formel I (Ia bzw. Ib) überführt.

Bevorzugte Abgangsgruppen sind Wasser und Essigsäure, wobei Wasser vorzugsweise durch katalytische Eliminierung mit Säuren, Essigsäure dagegen bevorzugt durch thermische Eliminierung bei 300°C abgespalten wird. Der Fortgang der Eliminierung kann anhand der abgeschiedenen Essigsäure bzw. des Wassers verfolgt werden. Bei der katalytischen Elimination verwendet man zweckmäßig ein Schleppmittel und fängt das entstehende Wasser in einem Wasserabscheider auf. Als Katalysatoren zur Wasserabspaltung haben sich Phosphorsäure und Triphenylphosphit bewährt; die Umsetzung dauert bei einer Temperatur von 180°C z.B. 1 - 8 Stunden. Sowohl die thermische als auch die katalytische Elimination führen bei Stilbenenfür die Ar je eine Phenylgruppe bedeuten, in einer Selektivität von ca. 90 % zum trans-Produkt. Zur Reinigung der Zielverbindung kann auf die Gel- bzw. Adsorptionschromtographie, die Destillation und/oder die Kristallisation zurückgegriffen werden.

Die in den nachstehenden Beispielen eingesetzten Alkyltoluole wurden nach Friedel-Crafts-Alkylierung aus Toluol und l-Alkenen hergestellt. Bei der Alkylierung wurden Isomerengemische erhalten, die nach $^1H$-NMR- und $^{13}C$-NMR-spektrokopischer Analyse aus ortho-, meta- und para-Alkyltoluolen mit skelletisomeren Alkylgruppen bestehen. Die Hauptprodukte sind para- und meta-Alkyltoluole mit in 2- oder 3-Stellung verzweigten Alkylketten. Diese Isomerengemischen wurden ohne weitere Trennung bei den Metallierungsreaktionen eingesetzt.

Die Lösungsmittel (Cyclohexan, Hexan, Tetrahydrofuran) wurden durch Destillation über n-Butyllithium und einer geringen Menge Styrol - als Indikator - von Verunreinigungen befreit.

Stickstoff wurde mit einer Mischung aus weißöl, 1 Gew.% Styrol und 5 Gew.% Lithiumbutyl gewaschen.

Die verwendeten Aldehyde wurden ohne zusätzliche Reinigung in der verfügbaren Reinheit von etwa 98 % eingesetzt.

Als Metallierungsmittel diente handelsübliches n-Butyllithium als 1,6 n-lösung in Hexan.

K-tert-butanolat wurde in der handelsüblichen Qualität mit einer Reinheit von 95 % verwendet.

Analytik:

a) Die Reinheit der Substanzen wurde mittels HPLC (HPLC-System Merck-Hitachi, UV-Detektor 655 A-22, Erma RI-Detektor ERC 7510, 4 mm Säule Lichrogel PS-4, THF als mobile Phase) überprüft.

Das Molekulargewicht der Katalysatoren wurde durch Korrelation zu HPLC-Eichungen mit Mono-, Di-, Oligo- und Polystyrol überprüft.

Herstellbeispiele 1 bis 12 für erfindungsgemäß verwendbare Stilbenderivate

Beispiel 1

Herstellung eines Stilbens der Formel

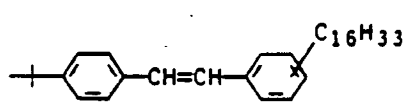

In einem Sechsliter-Glasreaktor, mit Kühl- bzw. Heizmantel- Bodenauslaß, Rührer aus Teflon und Kühler werden unter Stickstoffatmosphäre 95-4 g (0,85 mol) Kalium-tert.butanolat mit 850 ml Cyclohexan und 530 ml (0-85 mol) der Lösung von n-Butyllithium in Hexan versetzt. Zu dem Gemisch werden bei einer Temperatur von 20°C 269,2 g (0-85 mol) Hexadecyltoluol getropft. Die schwarz-braune Suspension wird für 16 Stunden bei 20°C gerührt und dann bei einer Temperatur von 10°C tropfenweise mit einer Lösung von 140 g (0,85 mol) 4-tert.-Butylbenzaldehyd in 100 ml Cyclohexan versetzt. Während der Zugabe (45 min) schlägt die Farbe von schwarz-braun nach gelb um.

Man rührt 30 Minuten nach und gibt dann 1,2 l In-Salzsäure zu. Die organische Phase wird abgetrenntmit Wasser neutral gewaschengetrocknet, i.V. von niedrig siedenden Bestandteilen befreit. Danach wird der Destillationsrückstand in einen 1-Liter-Kolben mit Wasserabscheider und Tropftrichter gebracht.

Als Katalysator für die Wasserabspaltung werden 10 ml Triphenylphosphit und als Schleppmittel 100 ml n-Octan zugesetzt. Man erhitzt auf 180°C und kontrolliert anhand der Menge des abgeschiedenen Wassers den Fortgang. Dann wird mit Wasser neutral gewaschen- getrocknet und fraktioniert destilliert. Bei einer Übergangstemperatur von 245 - 280°C unter einem Druck von 0,08 mbar erhält man 319 g (81,6 %) in einer Reinheit von 99,7 %.

Beispiel 2

Herstellung eines Stilbens der Formel

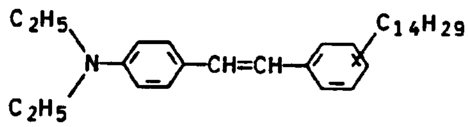

In einem Sechsliter-Glasreaktor, mit Kühl- bzw. Heizmantel, Bodenauslaß und Rührer aus Teflon werden unter Stickstoffatmosphäre 137 g (1,22 mol) K-tert.-butanolat, 1 l Cyclohexan und 320 g(1,11 mol) Tetradecyltoluol vorlegt und innerhalb von 25 Minuten mit 760 ml einer 1,6 n Lösung von (1,22 mol) n-Butyllithium in Hexan versetzt. Im Verlauf der Zugabe erwärmt sich die Reaktionsmischung auf 40°C. Man läßt 60 Minuten bei dieser Temperatur nachrühren, kühlt auf 10°C ab und tropft eine Lösung von 216 g (1,22 mol) 4-Diethylaminobenzaldehyd in 800 ml Cyclohexan so zu, daß die Innentemperatur stets im Bereich von 10 - 15°C bleibt. Während der Zugabe ändert sich die Farbe von schwarz-braun nach hell-gelb. Man läßt noch 30 Minuten nachrühren und tropft anschließend 260 g (2,4 mol) Essigsäureanhydrid zu. Das Reaktionsprodukt wird mit 2 l Wasser versetzt, die organische Phase abgetrennt und mit Wasser neutral gewaschen. Nach dem Abziehen des Lösungsmittels erhitzt man für ca. 2 Stunden auf 300°C und destilliert solange Essigsäure ab, bis sich nichts mehr abscheidet.

Dann wird fraktioniert destilliert. Man erhält 447 g (90 %) der Zielverbindung in einer Reinheit von 99,5 % bei einer Siedetemperatur von 270 - 298°C unter einem Druck von 0,5 mbar.

Beispiel 3

Wie in Beispiel 1 beschrieben, werden 247 g (0,78 mol) Hexadecyltoluol mit 0,86 mol Kalium-tert.-butanolat und 0,86 mol n-Butyllithium in 1000 ml Cyclohexan umgesetzt. Zu der schwarz-braunen Lösung tropft man bei 10°C 134 g (0,86 mol) 1-Naphthaldehyd. Die Reaktionsmischung wird 60 Minuten nachgerührt und mit 184 g (1,8 mol) Essigsäureanhydrid versetzt, wäßrig aufgearbeitet und destillativ von den leicht siedenden organischen Bestandteilen befreit. Zur Abspaltung der Essigsäure wird bei einem Druck von 100 mbar für 3 Stunden auf 300°C erhitzt. Anschließend wird im Ölpumpenvakuum fraktioniert destilliert. Man erhält bei 242-266°C/0,07 mbar 298 g (84 %) der Zielverbindung in einer Reinheit von 97 %. Nach anschließender Chromatographie in Kieselgel-S 0,063 - 0,2 mm mit einer Mischung aus 5 % Toluol und 95 % Cyclohexan als Eluens erhält man 251 g (73 %) der Zielverbindung in einer Reinheit von 98,6 %

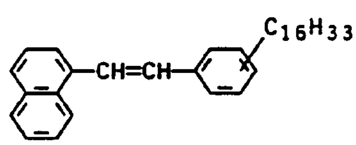

Die in der folgenden Tabelle aufgeführten Stoffe wurden auf entsprechend angepaßter Weise aus den sich jeweils ergebenden Vorprodukten hergestellt. Andere Stoffe dieser Art können leicht unter entsprechender Anwendung bzw Abänderung der Herstellvorschriften erhalten werden.

Tabelle 1

| Herstell-beispiel Nr. | Methylverbindung | Aldehyd | Equivalente n-BuLi/t-BuOK | Produkt |
|---|---|---|---|---|
| 1 | 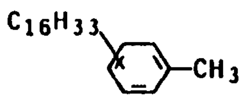 | 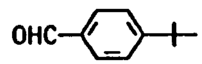 | 1.1 | 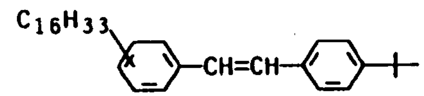 |
| 2 | 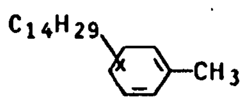 | 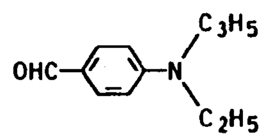 | 1.1 | 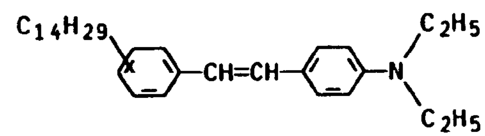 |
| 3 | 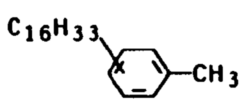 | 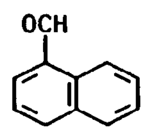 | 1.1 | 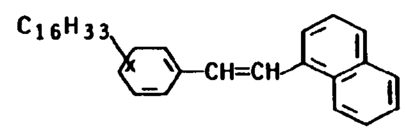 |
| 4 | 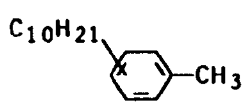 | 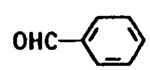 | 1.1 | 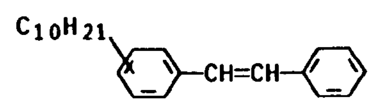 |
| 5 | 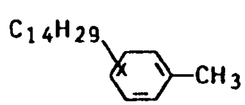 | 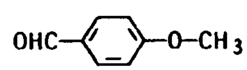 | 1.1 | 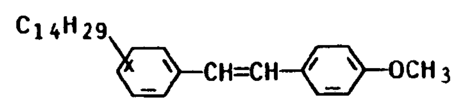 |

Tabelle 1 (Fortsetzung)

| Herstellbeispiel Nr. | Methylverbindung | Aldehyd | Equivalente n-BuLi/t-BuOK | Produkt |
|---|---|---|---|---|
| 6 | 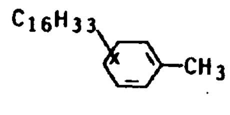 | 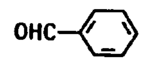 | 1.1 | 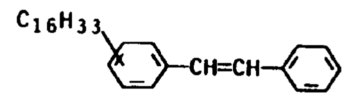 |
| 7 | 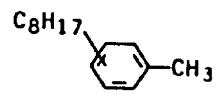 | 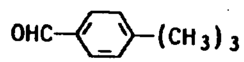 | 1.1 | 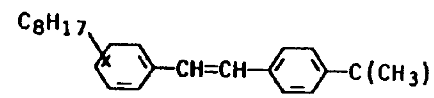 |
| 8 | 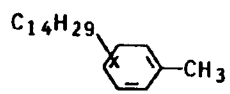 | 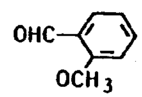 | 1.0 | 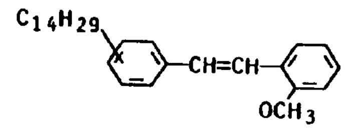 |
| 9 | 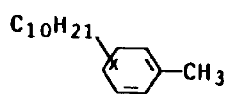 | 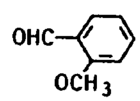 | 1.0 | 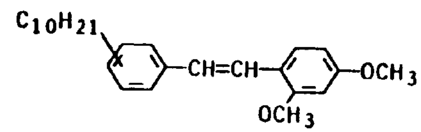 |
| 10 | 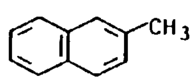 | 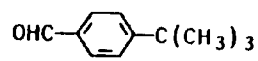 | 1.0 | 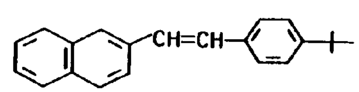 |

Tabelle 1 (Fortsetzung)

| Herstell-beispiel Nr. | Methylverbindung | Aldehyd | Equivalente n-BuLi/t-BuOK | Produkt |
|---|---|---|---|---|
| 11 | 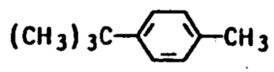 | 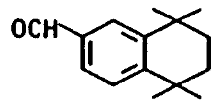 | 1.0 | 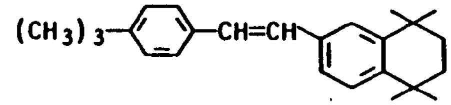 |
| 12 | 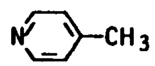 | 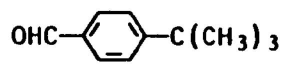 | 1.0 | 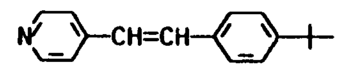 |

Tabelle 2

| Bsp.-Nr. | Rohausbeute nach Destillation | Reinausbeute nach Chromatographie (in Klammern Reinheit) | Sdp. Druck | eliminierte Gruppe * |
|---|---|---|---|---|
| 1 | 81 % (99,7 %) | -- | 245-280°C/0.08 mbar | W |
| 2 | 90 % (99,5 %) | 77 % (100 %) | 270-298°C/0.5 mbar | E |
| 3 | 84 % (97 %) | 73 % (98.6 %) | 242-266°C/0.1 mbar | E |
| 4 | 95 % (--) | 83 % (99 %) | 218-222°C/0.1 mbar | E |
| 5 | 79 % (98,2 %) | 68 % (99.5 %) | 261-270°C/0.2 mbar | E |
| 6 | 77 % (96,4 %) | 34 % (99.6 %) | 227-248°C/0.05 mbar | E |
| 7 | 73 % (--) | 63 % (99.0 %) | 217°C/0.05 mbar | W |
| 8 | 64 % (--) | 53 % (99.4 %) | 238°C/0.2 mbar | E |
| 9 | 63 % (98,9 %) | -- | 231-236°C/0.1 mbar | E |
| 10 | 75 % (99,0 %) | 65 % (99,0 %) | Smp. 130°C | E |
| 11 | 80 % (99 %) | -- | | E |
| 12 | 60 % (95 %) | -- | 180-202°C/1 mbar | E |

* W = Wasser
E = Essigsäure

Beispiel 13

Herstellung von

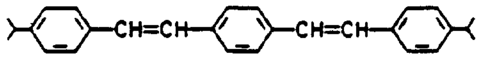

In einem 1 l Dreihalskolben mit Rückflußkühler und Tropftrichter werden unter Stickstoffatmosphäre 44,8 g (0,4 mol) Kalim-tert.-butanolat, 200 ml Cyclohexan und 53,6 g (0,4 mol) 4-Isopropyltoluol mit 250 ml einer 1,6 n Lösung von (0,4 mol) n-Butyllithium in Hexan versetzt. Man läßt 2 Stunden bei 60°C rühren- kühlt auf 10°C ab und tropft bei dieser Temperatur eine Lösung von 20,14 g (0,15 mol) Terephthaldialdehyd in THF zu. Die Lösung wird 30 Minuten bei Raumtemperatur nachgerührt und dann tropfenweise mit 1 mol Essigsäureanhydrid versetzt. Nach wäßriger Aufarbeitung werden die Lösungsmittel im Vakuum abgezogen und der Rückstand für 60 Minuten auf 300°C erhitzt. Durch Destillation im Ölpumpenvakuum werden 30 g (54 %) eines gelben Feststoffs erhalten, der zu 80 % aus der Zielverbindung besteht. Nach zweimaliger Kristallisation aus Ethanol erhält man 15 g (27 %) des gewünschten Produkts in einer Reinheit von 98,9 %.

Zur erfindungsgemäßen Verwendung der "Stilbene" Ia, Ib oder Ic ist folgendes zu sagen:

Es ist bekannt, alkalimetallorganische Verbindungen als Katalysatoren für die anionische Polymerisation von vorzugsweise Alkenylaromaten und/oder Dienen zu benutzen. Besonders gut geeignet sind Lithiumalkyle, da sie vergleichweise stabil sind und im Gegensatz zu den entsprechenden Natrium- oder Kaliumalkylen auch in Kohlenwasserstoffen löslich sind und in diesen Polymerisationen ermöglichen. Lebende Polymere mit Lithiumgruppen lassen sich in hohen Ausbeuten mit geeigneten Reagentien in endständig funktionalisierte Polymere umwandeln, z.B. in solche mit -OH, -SH oder Aminogruppen. Diese Reaktionen laufen am besten ab, wenn das Reaktionsmedium hauptsächlich aus Kohlenwasserstoffen besteht.

Während bekannte monofunktionelle Lithiumalkyle als Katalysatoren für die anionische Polymerisation alle Anforderungen erfüllen, weisen bekannte bifunktionelle Initiatoren Mängel auf. Solche Katalysatoren, insbesondere ätherfreie oder ätherarme, werden benötigt, wenn man an beiden Kettenenden funktionalisierte Polymere von Dienen erzeugen will, die diese vorzugsweise in 1,4-Konfiguration eingebaut enthalten.

Polyfunktionelle Initiatoren des Lithiums wurden z.B. hergestellt, indem man polyfunktionelle Halogenverbindungen mit metallischem Lithium in Äthern behandelt. Versucht man jedoch den Äther zu entfernen, so geht ein großer Teil der Aktivität verloren und die Katalysatoren werden unlöslich. Eine andere Herstellungsform ist die Addition von Lithium an kondensierte oder sonstige aromatische Ringsysteme wie z.B. Naphthalin, Biphenyl usw. oder an polyarylsubstituierte Ethylene wie 1,1-Diphenylethylen oder Stilben. Diese Reaktionen werden z.B. in der US-PS 3 170 903 beschrieben.

Auch diese Reaktion läßt sich nur in Ethern oder anderen polaren Lösungsmitteln realisieren. Die Gegenwart dieser Lösungsmittel führt jedoch bei der Polymerisation von Dienen zu Polymeren, die diese vorwiegend in 1,2- bzw. 3,4-Konfiguration eingebaut enthalten, weshalb solche Polymere weniger erwünschte Eigenschaften aufweisen, u.a. hohe Glastemperaturen- Oxydationsempfindlichkeit, Vernetzungsanfälligkeit und geringe thermische Stabilität bei der Verarbeitung.

Zur Vermeidung dieser Eigenschaften wurde vorgeschlagen, die Ether nach der Herstellung durch Abdestillation oder durch Fällung der Initiatoren mit Kohlenwasserstoffen zu entfernen. Das hat jedoch den Nachteil, daß die Initiatoren sich z. T. zersetzen. Auch sind Sie in Kohlenwasserstoffen unlöslich, so daß bei der Polymerisation unverhältnismäßig viel Initiatoren benötigt wird und Polymere mit breiter MGW-Verteilung entstehen.

Zur Überwindung dieser Nachteile wurde in der US-PS 3 377 404 von P. Zelinsky vorgeschlagen, die zunächst durch Umsetzung von Polyhalogenverbindungen, polykondensierten Aromaten oder polyarylierten Ethylenen mit Ethern entstehenden Dilithiuminitiatoren in einer 2. Reaktionsstufe durch Zusatz von Diolefinen zu solubilisieren und erst dann den Ether abzudestillieren.

Die entstehenden Katalysatoren sind zwar löslich in Kohlenwasserstoffen, jedoch werden die hierzu erforderlichen Diolefine praktisch ausschließlich in 1,2- bzw. 3,4-Konfiguration eingebaut. Außerdem tritt bei dieser Operation z. T. eine Zersetzung des Katalysators auf, so daß die hergestellten Polymere nur zum Teil mehrfunktionell sind.

Andere bekannte bifunktionellen Initiatoren werden durch Anlagerung von Li-Alkylen an 2 Doppelbindungen enthaltende Ausgangsverbindungen erzeugt. Infolge unvollständiger Reaktion oder ungenauer Dosierung enthalten sie häufig unerwünschte Anteile an monofunktionellen Katalysatoren. Auch sind sie häufig nicht ausreichend aktiv, so daß sie bei der Initiierungsreaktion unvollständig reagieren und/oder mit ihnen Polymere mit unerwünscht breiter MGW-Verteilung entstehen.

Gezielte Aufgabe der vorliegenden Erfindung war die Bereitstellung von auch in Medien, die ganz oder überwiegend aus Kohlenwasserstoffen bestehen löslichen, stabilen, hochaktiven, keine monofunktionelle Anteile enthaltenden bifunktionellen Alkalimetallinitiatoren, die Ausarbeitung eines für ihre Herstellung geeigneten Verfahrens, sowie die Verwendung dieser Katalysatoren für die Herstellung von an beiden Kettenenden gewachsenen- gegebenenfalls funktionalisierten Polymeren.

Diese Aufgaben werden erfindungsgemäß dadurch gelöst, daß man die nach dem vorstehend beschriebenen Verfahren erhältlichen, insbesondere substituierten Stilbene mit Alkalimetall, insbesondere Lithium reduziert und die erhaltene Bis-Alkalimetallverbindung als Katalysator verwendet.

Die einfachsten, für die Zwecke der anionischen Polymerisation geeigneten Stilbene haben die allgemeine Struktur

$$R^1,R^2,R^3Ar^1\text{-}CH{=}CH\text{-}Ar^2R^4,R^5,R^6 \qquad \text{(Ia)}$$

wobei $Ar^1$ und $Ar^2$ gleich oder verschiedene aromatische, auch polycyclische, gegebenenfalls Stickstoff enthaltende Ringsysteme bedeuten $Ar^1$ und $Ar^2$ können z.B. sein -Phenyl, -Naphthyl, -Diphenyl, Phenantryl-, Anthranyl-, Diphenylether-, Pyridyl-, Chinolylreste usw. Bevorzugt sind $Ar^1$ und $Ar^2$ Phenyl- oder Napthylreste.

$R^1$ bis $R^6$ (bzw. allgemein R) sind Wasserstoff oder lineare oder verzweigte Alkyl-, Alkenyl-, Aralkyl- oder Cycloalkylreste mit 1 bis 25 C-Atomen, die auch chemisch indifferente Substiuenten wie Etherbrücken oder tertiäre Aminogruppen enthalten können. Ferner können die Substituenten als Äquivalent auch ringförmige Brücken mit $Ar^1$ oder $Ar^2$ bilden, die jedoch mindestens 3 aliphatische C-Atome aufweisen müssen. Die Substituenten $R^1$ bis $R^6$ müssen zusammen jedoch mindestens 4 bis zu 60, vorzugsweise mehr als 8 bis zu 30 C-Atome enthalten.

Weniger als 4 C-Atome in den Substituenten erhöhen die Löslichkeit der Katalysatoren in Kohlenwasserstoffen nicht ausreichend. Wenn Dienpolymerisate oder Copolymere in Lösungsmitteln hergestellt werden sollen, die keine oder nur sehr geringe Mengen an Ethern enthalten (0 bis 3 Mol Ether je Mol polymerisationsaktives Lithium), sollten Initiatoren mit mehr als 8 C-Atome enthaltenden Substituenten Verwendung finden.

Bei den Substituenten $R^1$ - $R^6$ haben solche mit vorwiegend linearer oder wenig verzweigter Struktur eine bessere solubilisierende Wirkung als stark verzweigte Substituenten. So ist z.B. ein Katalysator aus Stilben, der an einen Benzolkern einen Decylrest trägt, besser in Kohlenwasserstoffen löslich als einer aus einem Stilben, das als $R^1$ eine t-Butylgruppe und $R^4$ und $R^5$ einen anellierten hydrierten Tetramethylcyclohexyl-Ring trägt (vgl. Tabelle 3, nachstehend). Die optimale Struktur ist für jeden Fall zu ermitteln.

Für die eingangs beschriebenen Verbindungen Ib und Ic gilt das vorstehend Gesagte sinngemäß.

Geeignete Verbindungen im Sinne der Erfindung sind z.B. im Kern alkylierte Derivate des cis- oder trans-Stilbens bzw. deren Gemische

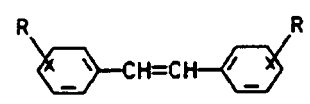

des 1-Phenyl-2 (1-8) Napthyl-Ethylens, des 1-Phenyl-2-(2-5-Pyridyl-Ethylens, sowie anderer Derivate des Ethylens wie

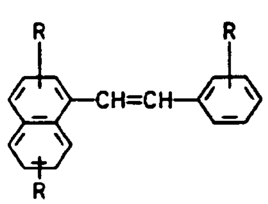

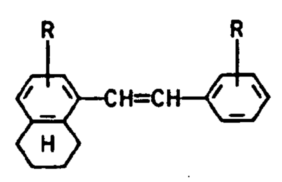

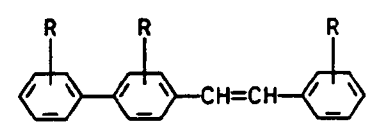

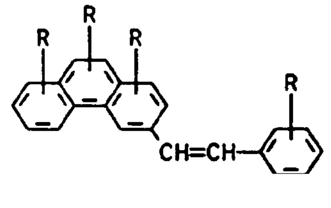

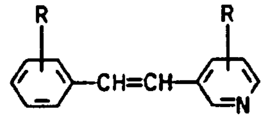

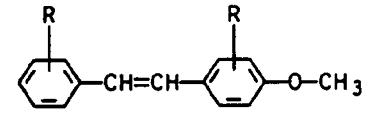

Ein eindrucksvolles Beispiel für eine solche - leicht herstellbare und mit Vorteil verwendbare - Verbindung ist

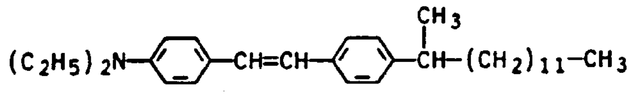

Diese Aufzählung ist nur beispielhaft und nicht vollständig. Besonders bevorzugt sind als $Ar^1$ und $Ar^2$ Phenyl- oder Napthylreste, $R^1$ bis $R^6$ lineare oder verzweigte Alkylreste, Cycloalkylreste,Dialkylaminoreste und/oder Äthergruppen mit zusammen 8 bis 30 C-Atomen, $R^1$ - $R^5$ Wasserstoff, z.B. Derivate des cis- oder trans-Stilben oder des 1-Phenyl-2-(1-Naphthyl)-Ethylens, die an den aromatischen Kernen einen bis 3, gegebenenfalls tertiären Stickstoff enthaltende aliphatische Substituenten tragen, die zusammen mehr als 8, aber weniger als 30 C-Atome enthalten.

Die unter Wärmeentwicklung ablaufende Metallierung der erfindungsgemäßen 1,2-Diarylethylene erfolgt, indem man diese in Gegenwart eines Ethers, eines tertiären Amins und wenn erforderlich- eines aliphatischen alicyclischen und/oder aromatischen Lösungsmittels bei einer Temperatur zwischen -20 - +70°C, vorzugsweise zwischen 0 und 40°C mit Alkalimetall umsetzt. Ein Vorteil der erfindungsgemäß zu verwendenden 1,2-Diarylethylene mit mehr als 4 C-Atomen in den Substituenten ist es, daß man bei der Umsetzung mit bereits vergleichsweise geringen Mengen an polaren Lösungsmittel in Gegenwart von größeren Mengen von Kohlenwasserstoffen vollständigem Umsatz erreicht. Die in Reaktionsgemisch enthaltene Menge an Ether oder tertiären Aminen sollte jedoch größer als 3 mol pro mol der substituierten 1,2-Diarylethylene sein, um ausreichende Reaktionsgeschwindigkeit und vollständigen Umsatz zu gewährleisten. Daneben können inerte Kohlenwasserstoffe in ähnlichen oder größeren Mengen im Reaktionsmedium enthalten sein. Für viele Aufgaben können solche Initiatoren unmittelbar ohne Entfernung des polaren Lösungsmittel verwendet werden, z.B., wenn man Dienpolymere anschließend hydrieren will und das Hydrierungsprodukt nicht kristallisieren soll.

Besonders gut geeignete aliphatische Ether sind z.B. Dimethylether, Diethylether, Dipropylether, Dibutylether, Diisopropylether, t-Butylmethylether usw., ferner auch alicyclische Ether wie Tetrahydrofuran usw. Die Reaktion läuft schon bei Zimmertemperatur zügig ab und ist häufig nach 15 bis 60 Minuten beendet, wobei mehr als 80 Gew.%, vorzugsweise mehr als 95 Gew.% der Ausgangsverbindungen umgesetzt werden. Benutzt man weniger gut geeignete aromatische Ether wie Anisol oder Phenetol, so dauert die Reaktion mehr als 10 mal so lange. Infolge unerwünschter Nebenreaktionen ist dann der Metallgehalt im Reaktionsmedium häufig erheblich höher als der Gehalt an Polymerisationsaktivität.

Als tertiäres Amin sind z.B. Trialkylamine, wie Triethyl-, Trimethyl- oder Dimethylethylamin usw. wie auch alicyclische oder aliphatisch/aromatische Amine wie N-Methylpiperidin oder Dimethylanilin geeignet.

Bei der Reaktion ist eine gute mechanische Durchmischung zweckmäßig. Lithium schwimmt z.B. infolge seiner niedrigen Dichte auf der Oberfläche des flüssigen Reaktionsmediums.

Nach der Metallierung kann man leichtflüchtige Ether oder Amine gegebenenfalls im Vakuum abdestillieren, wobei die Temperatur 70°, vorzugsweise 25°C nicht überschreiten sollte. Zweckmäßig sind dann neben den Ethern oder Aminen höher als diese siedende Kohlenwasserstoffe vorhanden. Niedrig siedende aliphatische Ether, z.B. Dimethylether, Diethylether, Diisopropylether, THF oder t-Butylmethylether oder niedermolekulare tertiäre Amine wie z.B. Triethylamin kann man dann z.B. ohne Schwierigkeiten bis auf Restgehalte von weniger als 0,1 mol Äther je mol polymerisationsaktives Lithium bringen. Die Löslichkeit der erfindungsgemäßen Katalysatoren bleibt, wenn die Substituenten $R^1$ - $R^9$ mehr als 8 C-Atome haben, meistens erhalten, solange mindestens 2 mol Ether je mol polymerisationsaktives Lithium Alkali vorhanden sind. Wird der Ether vollständig abdestilliert, so werden solche Katalysatoren, die aus Alkylstilbenen mit 4 bis 8 C-Atomen enthaltenden Substituenten hergestellt werden, schwer löslich und fallen teilsweise oder ganz aus.

Verwendet man solche Suspensionen zur Polymerisation, so gehen die Feststoffe nach einiger Zeit in Lösung. Es entstehen jedoch breitere Molekulargewichtsverteilungen. Katalysatoren aus Stilbenen mit langen, vorwiegend linearen oder nur leicht verzweigten Alkylgruppen und einem Gehalt von mehr als 8 C-Atomen in den Substituenten $R^1$ - $R^9$ bleiben gelöst. Die optimale Konfiguration hängt außerdem von den Resten Ar ab. Sie muß von Fall zu Fall ermittelt werden.

Lösungen von Katalysatoren, die wenig oder keine alicyclischen oder aliphatischen Ether bzw. solche, die nur aromatische Ether, wie Anisol oder Phenetol, oder tertiäre Aminen enthalten, bauen bei der Polymerisation Diene vorwiegend in 1,4-Konfiguration ein. Die entstehenden Polymeren haben meistens eine breitere Molekulargewichtsverteilung und höhere Molekulargewichte (durch GPC ermittelt), als bei Verwendung monofunktioneller Initiatoren, bei denen die experimentellen Molekulargewichte mit dem aus dem Verhältnis Monomer/Katalysator rechnerisch erwarteten relativ gut übereinstimmen.

Alle Lösungsmittel sowie die erfindungsgemäßen substituierten 1,2-Diarylethylene müssen vor der Verwendung von Verunreinigungen befreit werden. Geeignet ist z.B. die Destillation über ein Metallalkyl wie Aluminiumtriethyl unter Inertgasatmosphäre, z.B. unter trockenem, sauerstoffreiem Stickstoff oder Argon.

Bei der Herstellung der Katalysatoren finden kaum lithiumverbrauchende Nebenreaktionen statt. Je mol eingesetzten 1,2-Diarylethylens entsteht ca. 1 mol polymerisationsaktive Molekülzentren; diese sog. Polymerisationsaktivität, im folgenden PA genannt, läßt sich durch Titration der Lösung unter Inertbedingungen mit i-Propylalkohol bis zum Farbumschlag auf farblos ermitteln. Sie stimmt im allgemeinen gut überein mit dem Alkaligehalt der Lösung.

Bei der Reaktion der 1,2-Diarylethylene mit Alkalimetall entstehen 1,2-Dialkali-1,2-Diarylethan-derivate, die in Lösung intensiv gefärbt sind. Die Farbe liegt, je nach Ausgangsprodukt, zwischen dunkelbraun bis blauschwarz.

Die erfindungsgemäßen Katalysatoren sind hervorragend für die Polymerisation von Vinylaromaten, wie Styrol und dessen im Kern und/oder in $\alpha$-Stellung substituierten Alkylderivaten, sowie von Dienen wie Butadien, Isopren, 2,3-Dimethylbutadien, Piperylen und anderen geeignet. Die Polymerisation kann in allen Lösungsmitteln erfolgen, die auch für die Verwendung monofunktioneller Initiatoren benutzt worden. Aromaten wie Benzol oder Toluol sind als Lösungsmittel gut geeignet. Sie haben jedoch spezifische Nachteile wie Canzerogenität (Benzol) oder eine kettenübertragende Wirkung (Toluol), die aliphatische Lösungsmittel wie Cyclohexan oder Hexan usw. nicht haben.

Der Polymerisationsstart ist mit erfindungsgemäßen Katalysatoren im Vergleich zu monofunktionellen Katalysatoren etwas erschwert. Er wird erleichtert, wenn man Diolefine und Vinylaromaten mit gut in Kohlenwasserstoffen löslichen Spezies der erfindungsgemäßen Katalysatoren in Gegenwart geringer Mengen, z.B. 1 - 6 mol je mol PA, an Ethern oder tertiären Aminen polymerisiert. Es entstehen dann Polymere mit enger Molgewichtsverteilung und etwa dem Molekulargewicht, das man bei der amonischen Polymerisation mit monofunktionellen Initiatoren aus dem Verhältnis monomer/Initiator rechnerisch erwarten kann. In Gegenwart der Äther oder Amine werden Diene jedoch mit einem erhöhten Anteil von 1,2-Konfiguration in die Polymeren eingebaut. verzichtet man auf die Gegenwart von polaren Lösungsmitteln, um einen Einbau der Diolefine mit hohem Anteil an 1,4-Konfiguration zu erreichen, so ist die Initiierung mit den erfindungsgemäßen Initiatoren verlangsamt. Es entstehen bifunktionell wachsende Polymere mit breiterer MGW-verteilung und höherem als dem rechnerisch erwarteten Molekulargewicht. Manchmal werden auch 2 Polymerpeaks nebeneinander gemessen. Die MGW-Verteilung kann wieder enger gemacht werden durch Polymerisation in Gegenwart von 0,1 - 1 mol eines aliphatischen Li-Alkoholates je mol PA (US-P 4 754 329, EP-A-210 016). Dieser Zusatz beeinflußt die Konfiguration der eingebauten Diolefine nicht.

Mit bifunktionellen Katalysatoren kann man Blockcopolymere in weniger Stufen herstellen- als mit monofunktionellen Initiatoren. Polymerisiert man z.B. nacheinander Butadien und Styrol, so erhält man in 2 Stufen 3-Blockcopolymere, die in ihren Eigenschaften solchen Polymeren gleicher Zusammensetzung entsprechendie nach bekannten Methoden mit monofunktionellem Katalysator durch 3-stufige Polymerisation

Styrol → Butadien → Styrol

hergestellt wurden. Nach oxidativem Abbau des Polybutadienteiles der Polymeren mit Osmiumtetroxid (vgl. Angew. Makromol. Chem. 26, (1972), Seite 207) haben die verbleibenden Polystyrolblöcke in beiden Fällen das gleiche Molekulargewicht.

Die entsprechenden Natrium- oder Kaliumkatalysatoren sind in Kohlenwasserstoffen schwerer löslich. Dennoch läßt sich auch mit Ihnen z.B. Styrol in Cyclohexan oder anderen Kohlenwasserstoffen polymerisieren. Es entstehen jedoch breitere Molekulargewichtsverteilungen als mit Lithiumkatalysatoren.

Die Viskosität von mit erfindungsgemäßen Initiatoren hergestellten lebenden Polymerlösungen ist bei gleichem Molekulargewicht viel höher als die von mit monofunktionellen Katalysatoren hergestellten lebenden Polymeren, da die polaren, ionischen Kettenenden infolge Assoziation ein reversibles, physikalisches Netzwerk bilden. Die Assoziation ist um so höher, je weniger Ether im Lösungsmittelgemisch enthalten ist. Sie wird durch Umwandlung der carbanionischen Endgruppen mit Terminierungsreagentien in z.B. Li-Carboxylat-, Lithiumamid-, Lithiumalkoholat- oder lithiumthiolatendgruppen so verstärkt, daß bereits bei niedrigen Polymerkonzentrationen und Molgewichten Gelbildung unter Entstehen einer aspikähnlichen Masse eintritt. Mit großer Rührerenergie und hohem Drehmoment kann die Masse zur Erreichung vollständigen Umsatzes durchmischt werden. Wenn dieses Gel mit Wasser, Alkohol oder anderen, aktiven Wasserstoffe enthaltenden Verbindungen versetzt wird, so wird die ionische Vernetzung aufgehoben.

Die Viskosität der Lösung nimmt dabei um mehrere Größenordnungen ab.

Die Funktionalisierung der mit den erfindungsgemäßen Katalysatoren hergestellten, an beiden Kettenenden gewachsenen lebenden Polymeren ist in Abwesenheit oder Gegenwart von geringen Mengen polarer Lösungsmittel mit hohen Ausbeuten möglich. Reaktionen zur Funktionalisierung der lebenden Kettenenden sind bekannt. Geeignete Funktionalisierungsreagentien sind z.B. Oxirane, die endständige primäre oder sekundäre Hydroxylfunktionen liefern (vgl. US-Patent 3 786 116), oder Thiirane, mit denen endständige Thiolgruppen eingeführt werden können. Nach der EP-A-0 211 395 oder der europäischen Patentanmeldung 87 103 893.1 kann man Polymere erhalten, die am Kettenende mindestens eine Aminogruppe enthalten. Die Umsetzungen werden in den genannten Schriften eingehend beschrieben, so daß sich hier eine Beschreibung erübrigt. Sie kann z. T. den folgenden Beispielen entnommen werden.

Die erfindungsgemäßen Polymeren können, wenn sie ganz oder zum Teil aus Dienen aufgebaut wurden, hydriert werden, wobei die aliphatischen Doppelbindungen ganz oder zum Teil verschwinden. Die Hydrierung wird mit Hilfe von molekularem Wasserstoff und Katalysatoren auf Basis von Metallen oder Metallsalzen der 8. Nebengruppe des Periodensystems durchgeführt, entweder in homogener oder heterogener Phase. Die Verfahren sind bekannt und werden z.B. in der US-Patentschrift 3 113 986, der DE-B 1 222 266, DE-A 2 013 263, DE-B 1 106 961 oder DE-A 1 595 345 beschrieben.

An beiden Kettenenden mit Mercapto-, Hydroxyl- oder Aminogruppen funktionalisierte Polymere sind als Präpolymere für Polyurethane, Epoxidharze und andere Harze oder für deren Modifizierung von besonderem Interesse. Die Herstellung von Epoxidharzen und von elastomeren Polyurethanen, bestehend aus einem "Hartsegment" aus aromatischen Polyisocyanaten und einem "Weichsegment" aus funktionalisierten flexiblen Makromolekülen ist bekannt und wird von H.P. Elias, in der Publikation Makromoleküle, Seiten 778 - 780 und 809 - 812, 4. Auflage. (1981), Hüttig und Wepf Verlag, Basel-Heidelberg-New York und der dort zitierten Literatur beschrieben.

Erfindungsgemäß aus Dienen und/oder auch aus Vinylaromaten hergestellte funktionalisierte Polymere, die an den Kettenenden Amino- oder Hydroxylgruppen enthalten, können z.B. mit Diisocyanaten und anderen Reagentien vernetzt werden. Lösungen solcher Polybutadiene, versetzt mit Diisocyanaten, ausgegossen auf silikoniertes Papier und getrocknetergeben bei überwiegend aus Dienen aufgebauten Polymeren elastische, trockene, in Kohlenwasserstoffen unlösliche Filme, die sich von der Unterlage abziehen lassen und hohe reversible Dehnungen aufweisen.

Polybutadiendiole zeichnen sich als "Weichsegmente" in thermoplastischen Polyurethanen durch besonders gute Entmischung von "Hart- und Weichsegmenten" aus, was aus anwendungs- und verarbeitungstechnischen Gründen erwünscht ist, wie dies auch von Becker und Braun, Kunststoffhandbuch Band 7, Polyurethane, Seite 33 (1983), 2. Auflage Hanser Verlag, München-Wien, beschrieben wird. Bei gleichem Molekulargewicht aus dem Gewichtsmittel weisen solche erfindungsgemäß hergestellten Öle wegen ihrer engen Molekulargewichtsverteilung eine niedrigere Viskosität auf als bekannte Präpolymere, wie z.B. radikalisch hergestellte telechele Polybutadienöle- Polytetrahydrofuran oder Polyester. Sie sind daher besser verarbeitbar.

Erfindungsgemäß erhaltene Polymere weisen im allgemeinen Durchschnittsmolekulargewichte (Gewichtsmittel Mw) zwischen 500 und 500 000, vorzugsweise 3 000 und 130 000 auf, ermittelt durch Gelpermeationschromatographie (GPC) und Vergleich mit standardisierten, zur Eichung geeigneter Polymeren (vgl. G. Glöckner, "Polymercharakterisierung durch Flüssigkeitschromatographie", verlag A. Hüthig, Heidelberg, (1982). Gemessen wird in 0-25 gew.%iger Tetrahydrofuranlösung bei 23°C und einer Durchflußgeschwindigkeit von 1,2 ml/min. Das Molekulargewicht wird zweckmäßig vor der Funktionalisierung ermittelt, da manche funktionalisierte Polymere von GPC-Säulen adsorbiert werden und diese unbrauchbar machen.

Die Aufarbeitung der Polymeren erfolgt auf übliche Weise, z.B. durch Fällen mit einem Nichtlösungsmitteldurch Abdampfen des Lösungsmittels, oder durch Wasserdampfdestillation. Auch Entgasung auf einen Entgasungsextruder ist möglich.

Die nachstehend verwendeten Stilbenderivate wurden auf die oben beschriebene Weise hergestellt, können aber zu Versuchszwecken auch nach anderen bekannten Methoden der organischen Chemie hergestellt werden.

In den Beispielen wurden die folgenden substituierten 1,2-Diarylethylene als Ausgangsprodukte benutzt, die zum überwiegenden Anteil in Trans-konfiguration vorlagen.

Tabelle 3

| Bsp. | Struktur der 1,2-Di-arylethylene | FP | KP | Reinheit laut HPLC | Herstellung |
|---|---|---|---|---|---|
| A | 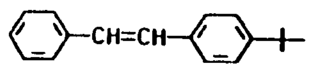 | 89-99°C | -- | 97,47 % | Benzylmagnesiumchlorid + p-t-Butyl-Benzaldehyd → Dehydratisierung → Umkristallisieren |
| B | 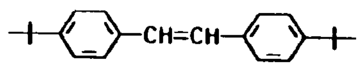 | 173-176°C | 187-213°C 0,08 mbar | 97,45 % | t-Butyl-benzylmagnesiumchlorid + p-t-Butylbenzaldehyd → Dehydratisierung → Umkristallisieren |
| C | 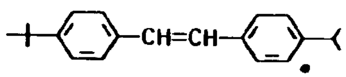 | 118-121°C | 174-184°C 0,04 mbar | 99,48 % | p-t-Butylbenzylmagnesiumchlorid + p-Isopropylbenzaldehyd → Dehydratisierung → Umkristallisieren |
| D | 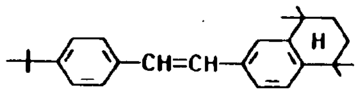 | flüssig | -- | 99,2 % | p-t-Butylbenzylmagnesiumchlorid + 5,6,7,8-tetrahydro-5,5,8,8-tetramethylnaphthaldehyd-2 → Umkristallisieren |

Tabelle 3 (Fortsetzung)

| Bsp. | Struktur der 1,2-Di-arylethylene | FP | KP | Reinheit laut HPLC | Herstellung |
|---|---|---|---|---|---|
| E | 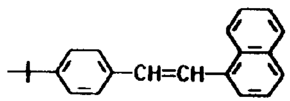 | 90-91°C | -- | 99,62 % | p-t-Butylbenzylmagnesiumchlorid + 1-Naphtaldehyd → Dehydratisierung → Umkristallisieren |
| F | 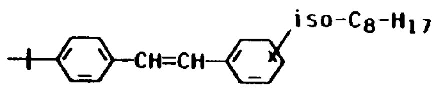 (60 % m-, 40 % p-Verb.) | 82-86°C | 217-225°C 0,05 mbar | 98,97 % | Isooctylbenzyllithium - p-t-Butylbenzaldehyd → Dehydratisierung |
| G | 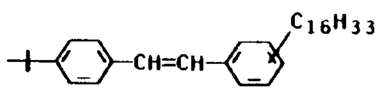 (n - iso, 60 % m-, 40 % p-Verb.) | flüssig | 284-300°C 0,07 mbar | 99,68 % | n - iso-Hexadecylbenzyllithium → t-Benzaldehyd → Dehydratisierung |
| H | 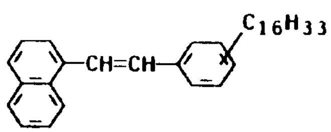 (n - iso, 60 % m-, 40 % p-Verb.) | flüssig | 242-266°C 0,11 mbar | 98,69 % | Hexadecyl-benzyllithium + 1-Naphtaldehyd → Dehydratisierung |

Tabelle 3 (Fortsetzung)

| Bsp. | Struktur der 1,2-Di-arylethylene | FP | KP | Reinheit laut HPLC | Herstellung |
|---|---|---|---|---|---|
| I | 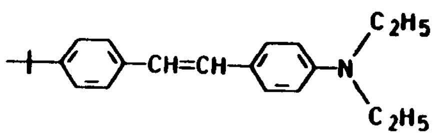 | 125-126°C | 175-187°C 0,05 mbar | 99,18 % | p-t-Butylmagnesium-chlorid + p-Diethylaminobenzaldehyd → Acetylierung → Thermolyse bei 280 - 300°C |
| J | 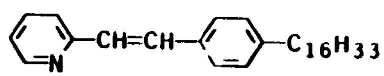 (n - iso, 60 % m-, 40 % p-Verb.) | flüssig | 285-305°C 0,05 mbar | 73,62 % | Hexadecyl-benzyl-lithium + 2-Pyridinaldehyd → Acetylierung → Thermolysierung |
| K | 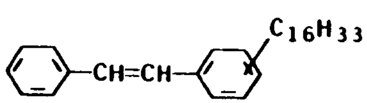 (n - iso, 60 % m-, 40 % p-Verb.) | flüssig | 227-248°C 0,05 mbar | 99,58 % | n + iso-Hexadecyltoluollithium + Benzaldehyd → Acetylierung → Thermolyse |

Tabelle 3 (Fortsetzung)

| Bsp. | Struktur der 1,2-Di-arylethylene | FP | KP | Reinheit laut HPLC | Herstellung |
|---|---|---|---|---|---|
| L | 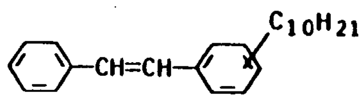 (n - iso, 60 % m-, 40 % p-Verb.) | flüssig | 218-222°C 0,10 mbar | 98,19 % | n + iso-Decyltoluol-Li + Benzaldehyd → Acetylierung → Thermolyse |
| M | 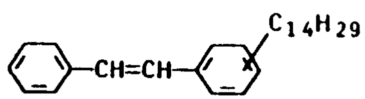 (n - iso, 60 % m-, 40 % p-Verb.) | flüssig | 230-238°C 0,2 mbar | 98,37 % | n - iso-Tetradecyltoluollithium + o-Methoxybenzaldehyd → Acetylierung → Thermolyse |
| N | 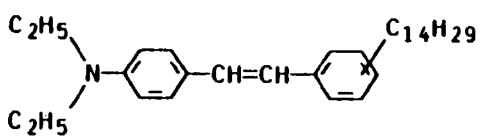 (n - iso, 60 % m-, 40 % p-Verb.) | flüssig | 270-298°C 0,45 mbar | 100 % | n - iso-Tetradecyltoluollithium + p-Diethylaminobenzaldehyd → Acetylierung → Thermolyse |

Die Lösungsmittel (Benzol, Cyclohexan, Methylcyclohexan, Octan, Tetrahydrofuran, Diethylether, Dibutylether, Diisopropylether, Triethylamin) wurden durch Destillation mit soviel Butyllithium und einer geringen Menge als Indikator dienenden Styrols, daß eine bleibende Orangenfarbe blieb- von Verunreinigungen befreit.

Der bei -25°C siedende Dimethylether wurde einer Druckflasche entnommen, in einer mit sec.-Butyllithium-Methylcyclohexanlösung beschickten Waschflasche gereinigt und gasförmig in den Reaktor gegeben.

Stickstoff wurde mit einer Mischung aus Weißöl, 1 Gew.% Styrol und 5 Gew.% Lithiumbutyl gewaschen.

Lithium wurde in Form eines 1 bis 5 mm großen Granulates der Firma Merck-Schuchardt verwendet.

Natrium wurde in Form einer Legierung mit Kalium im Mol-verhältnis 1:1 verwendet.

Für die Polymerisationsversuche wurden handelsübliches Butadien und Styrol eingesetzt. vor der Verwendung wurden die Monomeren durch Destillieren nach Zusatz von 0,5 Gew.% Triisobutylaluminium gereinigt.

Für die Funktionalisierung wurde 1,5-Diazo-bicyclo(3,1,0)hexan, gemäß europäischer Patentanmeldung 87 103 893, im folgenden Propylendiaziridin genannt, sowie handelsübliches Ethylenoxid, das einer Stahlflasche entnommen wurde verwendet.

Analytik:

a) Charakterisierung der erfindungsgemäßen Katalysatoren

aa) Bestimmung der Polymerisationsaktivität (PA)

Unter PA wird der Gehalt an polymerisationsaktivem Lithium verstanden 1 mol difunktioneller Katalysator entspricht also 2 mol PA.

Aus dem Reaktor oder dem Vorratsgefäß werden mit einer kalibrierten 5 ml Einwegspritze aus Polypropylen (Hersteller Fa. Braun-Melsungen AG, BRD), die mit einer 15 cm langen Kanüle versehen ist, unter inerten Bedingungen (die Spritze wird durch mehrfaches Aufziehen und Entleeren von Verunreinigungen befreit) eine ca. 2 - 3 mmol PA enthaltende Menge an Lösung entnommen und durch eine Gummikappe in einen mit Reinstickstoff gespülten 250 cm-Kolben gegeben, in dem 100 ml Toluol mit einem Magnetrührer gerührt wird. Verunreinigungen im Toluol wurden zuvor mit der Reaktionslösung bis auf schwache Gelbfärbung austitriert. Die intensiv gefärbte Lösung wird dann mit einer 5 $cm^3$ fassenden kalibrierten Injektionsspritze mit 1 n Isopropanol in absolutem Toluol auf farblos titriert. Die Gesamtmenge an PA im Reaktor errechnet sich dann aus

$$\frac{\Sigma \text{ Vorrat } + \text{ Entnahme } (cm^3)}{cm^3 \text{ Entnahme}} \text{ x } cm^3 \text{ 1 n i - Propanol } = \text{ mmol PA}$$

ab) Bestimmung des Alkaligehaltes

Wie unter aa) beschrieben, wird eine ca. 1 mmol PA entsprechende Menge Katalysatorlösung entnommen und unter Stickstoff in eine Mischung aus 10 $cm^3$ Cyclohexan und 1 $cm^3$ Methanol gegeben. Die farblose Mischung wird dann mit 20 $cm^3$ destilliertem Wasser ausgeschüttelt. Nach dem Absetzen wird der klare wäßrige Extrakt mit einer Injektionsspritze entnommen und die Lösung noch 2 x mit je 10 $cm^3$ Wasser extrahiert. Die vereinigten Extrakte werden in einem Erlenmeyerkolben so lange gekocht, bis die organischen Lösungsmittel entfernt sind. Nach Abkühlen wird mit n/10 HCL und Phenolphthalein auf neutral titriert.

$$\frac{\Sigma \text{ Vorrat } + \text{ Entnahme } (cm^3)}{cm^3 \text{ Entnahme x 10}} \text{ x } cm^3 \text{ n/10 HCL } = \text{ mmol Alkali}$$

ac) Bestimmung der im Katalysator enthaltenen Ether bzw. tertiären Amine.

5 $cm^3$ der Katalysatorlösung, die ca. 1 bis 3 mmol PA enthalten, werden in einem 25 $cm^3$ fassenden Destillationskolben unter Stickstoff mit X $cm^3$ einer Lösung von 1n-Isopropanol in Toluol bis zur Entfärbung titriert. Das gesamte Lösungsmittel wird dann in eine mit Methanol-/Kohlensäuremischung gekühlte Vorlage übergetrieben und im Destillat der Gehalt an Ethern bzw. Aminen in Gew.% durch Gaschromatographie bestimmt. Es wurde ein Gaschromatograph der Fa. Shimadzu GC-3BT mit der Säule Carbowax 20M, bei 60°C benutzt.

Das Molverhältnis Ether/Polymerisationsaktivität (PA) ergibt sich aus

$$\frac{\text{Gew.\% Ether/tert. Amine x } (5 + X) \text{ x} m^3}{\text{mmol PA x 7,2}} = \frac{\text{mmol Ether/tert. Amine}}{\text{PA}}$$

b) Charakterisierung der erfindungsgemäßen Polymerisate

ba) Bestimmung des Molekulargewichts (MGW) durch GPC

Zur Bestimmung kamen die nicht funktionalisierten Proben zur Anwendung. Gemessen wurde mit einem GPC-Gerät der Fa. Waters. Die Molgewichte wurden an Hand von Eichkurven durch Vergleich mit standardisierten, zur Eichung geeigneten Polymeren bestimmt (vgl. G. Glöckner, "Polymercharakterisierung durch Flüssigkeitschromatographie", Verlag A. Hüthig, Heidelberg, 1982). Gemessen wurde in 0,25 %iger Lösung in THF bei 23°C und einer Durchlaufgeschwindigkeit von 1,2 $cm^3$/Min.

Bei Blockcopolymeren wurde als MGW empirisch das der Zusammensetzung entsprechende arithmetrische Mittel zwischen den Eichkurven der beiden Homopolymeren zugrundegelegt.

bb) Bestimmung der Viskositätszahl

Die Viskositätszahl (VZ) wurde bei 25°C in Toluol (0,5 g Polymer in 100 cm3 Toluol) gemäß DIN 51562 bestimmt.

bc) Bestimmung des Stickstoffgehaltes

Der Gesamtstickstoffgehalt wurde nach Kjeldahl bestimmt.

bd) Bestimmung der mechanischen Eigenschaften

Die mechanischen Daten (Zugfestigkeit bei 300 % Dehnung, Reißfestigkeit und Reißdehnung) wurden bestimmt an Prüfkörpern, die nach DIN 53455 aus zwischen Teflonscheiben gepreßten 2 mm starken Platten (Preßtemperatur 170 bis 180°C, 60 bar) oder Folien (Preßtemperatur 150°C, 10 bar) ausgestanzt werden.

be) Vernetzung mit Diisocyanat

5 g des Polymeren wurden in 25 $cm^3$ trockenem Cyclohexan gelöst und mit 0,33 mmol einer Lösung von Toluylendiisocyanat (TDI) in Cyclohexan versetzt. Nach Durchmischung wurde auf silikoniertes Papier gegossen und bei Zimmertemperatur getrocknet.

bf) Abbau von Butadien/Styrolblockcopolymeren und Bestimmung des Molgewichts der Polystyrolblöcke

Das Verfahren des oxidativen Abbaus des Polybutadienteiles mit Peroxid/Osmiumtetroxid wird von Ph. Kubin-Eschger, Angew. Makromol. Chem. 26, (1972), Seite 207 detailliert beschrieben, so daß sich eine weitere Beschreibung erübrigt.

bg) Bestimmung der OH-Zahl

Zur Bestimmung der OH-Zahl wurden die umgefällten Polybutadienöle zunächst in der Schmelze bei 140°C und einem Vakuum von 0,4 mbar so lange gerührt, bis alle flüchtigen Anteile entfern waren und keine Gasentwicklung mehr zu beobachten war. Ca. 2 g des Öles wurden unter Reinststickstoff in einem 250 $m^3$ Kolben in hochgereinigtem Cyclohexan gelöst und mit 50 $cm^3$ THF gemischt. Die Lösung wurde nach Zugabe von 0,5 $cm^3$ als Indikator dienendem 1,1-Diphenylethylen mit einer 0,1 n Lösung von n-Li-Butyl in Cyclohexan bis zum Auftreten einer schwach orange-Färbung austitriert. Die Titration erfolgte durch eine Gummikappe mit einer kalibrierten Spritze. vom ermittelten Verbrauch wurde der in gleicher Weise ermittelte Blindwert abgezogen.

Die Methode war zuvor durch $^1$H-NMR-Spektroskopie auf ihre Brauchbarkeit geprüft worden.

In den $^1$H-NMR-Spektren der OH-terminierten Polybutadiene erscheinen die Methylenprotonen der $-CH_2-$OH-Gruppe als deutlich separierte Absorption im Bereich von = 3,6 ppm. Aus dem Intensitätsverhältnis dieser Resonanzen im Vergleich zu den Absorptionen der Polybutadien-Hauptkette kann der Gehalt an OH-Gruppen, ausgedrückt als OH-Zahl, errechnet werden. Die Arbeitsmethodik kann z.B. dem Buch "Spektroskopische Methoden in der organischen Chemie" von Manfred Hesse, Herbert Meier, Bernd Zeck, Georg Thieme Verlag, Stuttgart - New York, 3. Auflage 1987, entnommen werden. vgl. auch z.B. Pol. J. Vol. 17, No. 8, pp. 977-980 (Short Comm.).

Beispiele 14 - 29

In den Beispielen 14 - 29 wird die Herstellung erfindungsgemäßer Katalysatoren beschrieben. Als Reaktor dient ein mit Magnetrührer (ohne Teflonummantelung) und Thermometer ausgerüsteter 500 $cm^3$ Vierhalskolben, der einen mit einer Gummikappe verschlossenen Stutzen besitzt und mit Reinstickstoff gespült werden kann. Der Reaktor ist in ein kühlbares Wasserbad eingebaut.

Der Reaktor wird jeweils mit hochgereinigtem Ether oder tertiärem Amin in einer Menge entsprechend dem in der Tabelle 4 angegebenen Molverhältnis sowie gegebenenfalls Methylcyclohexan oder anderen inerten Kohlenwasserstoffen und ca. 2,0 g handelsüblichen Lithiumgranulates bestückt. Dazu werden 50 mmol des substituierten 1,2-Diarylethylen gegeben. Die Reaktion setzt bei 25°C, je nach Reinheit der Produkte sofort oder nach bis zu 15 Minuten, unter Färbung des Inhaltes und Temperaturerhöhung ein. Es wird so mit Wasser gekühlt- daß 25°C nicht überschritten werden. Die Reaktion ist beendet, wenn Innen- und Badtemperatur wieder übereinstimmen, im allgemeinen nach 30 Minuten bis eine Stunde. In der Lösung werden PA und Li-Gehalt bestimmt. Bei den in Tabelle 4 mit a) gekennzeichneten Versuchen wurde ein 50 mmol PA enthaltender Teil der Katalysatorlösung (ca. 50 $mm^3$) in einen 250 $mm^3$ Destillationskolben überführt, auf dem eine 30 cm lange mit Sulzermetallpackungen gefüllte isolierte Kolonne mit 20 mm $\phi$ sitzt. Der Rückflußkühler wird mit Sole von -20°C gekühlt. Die Destillationsapparatur wurde zuvor zur Reinigung mit einer Li-Butyllösung ausgekocht. Nach Zugabe von 100 $mm^3$ Methylcyclohexan wurde der Äther bzw. das THF aus der Katalysatorlösung bei allmählich zunehmendem Vakuum bei einem Rücklaufverhältnis von ca. 1:10 so abdestilliert, daß die Temperatur im

Sumpf 20 - 25°C nicht überschreitet. Es wird so lange abdestilliert, bis die Temperatur am Übergang und im Sumpf gleich sind.

In der Tabelle 4 sind der Gehalt an Polymerisationsaktivität (PA) und Lithium in % der Theorie (Umsetzungsgrad), Farbe und Konsistenz der Katalysatoren und weitere relevante Daten zusammengestellt.

Tabelle 4 (54)

| Beispiel Nr. | SDAE [1] (Nr. aus Tab. 1) | Polares Lösungsmittel (PL) | | Unpolares Lösungsmittel | Umsetzungsgrad in % d. Theorie | | Katalysatorlösung | | |
|---|---|---|---|---|---|---|---|---|---|
| | | Name | Molverh. PL/SDAE | | PA | Li-Gehalt | Konzentration Mol PA/l | Farbe | Konsistenz |
| 14 | A | THF | 20 | -- | 98,4 | -- | 0,62 | • | Lösung |
| 14a [3] | A | THF | 1 | Methylcyclohexan | 95 | 95 | 0,34 | • | dicke Suspension (Pudding) |
| 15 | A | DMEA [4] | 9 | Benzol | 100 | 99 | 2,28 | • | dicke Suspension (Pudding) |
| 16 | B | THF | 10 | Methylcyclohexan | 95 | 98 | 0,52 | • | Lösung |
| 16 [3] | B | THF | 1,54 | Methylcyclohexan | 100 | 93 | 0,25 | • | Suspension |
| 17 | B | THF | 3 | Methylcyclohexan | 85 | 92 | 0,87 | • | Suspension |
| 18 | C | THF | 10 | Methylcyclohexan | 94 | 92 | 0,43 | • | Lösung |
| 18a [3] | C | THF | 1,5 | Methylcyclohexan | 96 | -- | 0,3 | • | Suspension |
| 19 | D | THF | 10 | Methylcyclohexan | 96 | 96 | 0,47 | • | Lösung |
| 19a [3] | D | THF | 1,27 | Methylcyclohexan | 100 | 100 | 0,4 | • | Suspension |
| 20 | E | THF | 6 | -- | 97,5 | 100 | 2,0 | Blauschwarz | Lösung |
| 21 | E | THF | 3 | Methylcyclohexan | 80 | -- | 1,5 | • | Suspension |
| 22 | I | THF | 6 | -- | 106 | 100 | 2,2 | Braunschwarz | Lösung |
| 22a [3] | I | THF | 1,5 | Methylcyclohexan | 100,75 | 97,5 | 2,2 | • | Suspension (Pudding) |
| 23 | G | DEE [5] | 2 | Methylcyclohexan | 100,5 | 97 | 2,5 | • | Lösung |
| 23a | G | DEE [5] | 0 | Methylcyclohexan | 106 | 99,8 | 1,2 | • | Lösung |
| 24 | F | THF | 2 | Methylcyclohexan | 99 | 101 | 2,36 | • | Lösung |
| 25 | J | DEE [5] | 6 | Methylcyclohexan | 99,2 | 92 | 1,0 | Schwarzbraun | Lösung |
| 26 | K | DEE [5] | 4 | Methylcyclohexan | 104,4 | 100,1 | 1,2 | • | Lösung |
| 26a [3] | K | DEE [5] | 0,09 | Methylcyclohexan | 100 | 100 | 0,4 | • | Lösung |
| 27 | M | DEE [5] | 4 | Methylcyclohexan | 99,6 | 99 | 1,2 | Schwarzbraun | Lösung |
| 27a [3] | M | DEE [5] | 0,03 | Methylcyclohexan | 104,4 | 99 | 0,8 | • | Lösung |
| 28 | N | DEE [5] | 4 | Methylcyclohexan | 101,6 | 97 | 1,1 | • | Lösung |
| 28a [3] | N | DEE [5] | 0,17 | Methylcyclohexan | 100 | 101,5 | 0,45 | • | Lösung |
| 29 | L | DEE [5] | 4 | Methylcyclohexan | 101 | 99 | 1,2 | • | Lösung |
| 29a [3] | L | DEE [5] | 0,0 | Methylcyclohexan | 100 | 99 | 0,8 | • | Lösung |

1) SDAE = substituiertes Diarylethylen
2) Theorie = 2 Mol je Mol SDAE
3) PL im Vakuum abdestilliert
4) Dimethylethylamin
5) Diethylether

Beispiel 30

Herstellung eines Dinatriumkatalysators

Es wird verfahren wie in Beispiel 29, jedoch wird zur Dimerisierung mit 13 g einer flüssigen, Natrium und Kalium im Molverhältnis 1:1 enthaltenden Legierung gearbeitet. Als Alkenylaromat wurde das Diaryl-1,2-ethylen K aus Tabelle 3 verwendet. Die Reaktion ist nach 30 Minuten beendet, die Umsetzung war vollständig. Die schwarz-braune Lösung enthielt 100 mmol PA, und 99 mmol Alkali, die Konzentration war 1,2 mol PA/l.

Beispiel 31

Herstellung eines etherarmen Katalysators Mit Dimethylether (DME)

In den Reaktor von Beispiel 1 wurden 14 $cm^3$ Methylcyclohexan, 50 mmol SDAE Nr. L aus Tabelle 3 gegeben und gereinigtes Dimethylethergas über die gut durchmischte Flüssigkeit geleitet. Die Reaktion setzt nach 5 Minuten unter Anstieg der Temperatur von 21,5° auf 23,5° ein und ist nach einer Stunde vollständig. Es werden 98,4 mmol PA und 97,4 mmol lithiumgehalt gemessen.

Der Reaktorinhalt wird unter Spülen mit 10 $cm^3$ Methylcyclohexan in den Destillationskolben überführt und unter Rühren bei zuletzt 10 torr 40 $cm^3$ des Kolbeninhaltes bei Innentemperatur um 0°C abdestilliert. Nach Aufheben des Vakuums wird die Lösung mit 40 $cm^3$ Methylcyclohexan verdünnt, unter Nachwaschen in ein unter Reinstickstoff stehendes Vorratsgefäß überführt und auf 150 $cm^3$ aufgefüllt. Konzentration 0-65 mol PA/l.

Polymerisationsversuche

Beispiele 32 - 44

Für den Polymerisationsversuch dient als Reaktor ein mit einem Rührer, einem mit Sole von -30°C betriebenem Sole-Rückflußkühler, einem kalibrierten, ebenfalls mit Sole-Rückflußkühler versehenem Tropftrichter, einem mit einer Gummikappe verschlossenen Stutzen sowie einer Reinstickstoffspülung versehener 10 l-Glaskolben mit Heiz- bzw. Kühlmantel. Der Stickstoff wird durch Waschen mit einem 2 Gew.% Lithiumbutyl enthaltenden Weißöl von Spuren von Feuchtigkeit und Sauerstoff befreit.

Der Reaktor wird zunächst mit einer Lösung von Lithiumbutyl in Cyclohexan, der etwas Styrol zugesetzt wurde, ausgekocht. Die als Indikator für die Aktivität der Lösung dienende orangene Farbe muß bis zum Schluß vorhanden sein. Die Lösung wird abgezogen und der Reaktor mit 3 l Cyclohexan befällt, das zuvor über eine Säule mit Molekularsieb gereinigt wurde. Die noch vorhandenen Varunreinigungen werden bei 40°C mit einer erfindungsgemäßen Katalysatorlösung durch die Gummikappe mit einer kalibrierten Spritze bis zum Auftreten eines geringen Orangetones austitriert.

In den Beispielen wurden 3-Blockcopolymere S-B-S der Zusammensetzung 50 bzw. 27 Gew.% Styrol, 50 bzw. 73 Gew.% Butadien mit dem Zielmolekulargewicht 20000 bzw. 60000 hergestellt. Dazu wurden in den Reaktor nach dem Austitrieren bei den in der Tabelle 5 angegebenen Starttemperaturen ca. 50 $cm^3$ Butadien und die erforderliche Menge der jeweiligen Katalysatoren (27 bzw. 9 mmol PA) gegeben, und nach Beginn der Polymerisation das restliche Butadien (insgesamt 215 $cm^3$ = 134,5 g bzw. 300 $cm^3$ = 187,5 g) bei ca. 70°C zulaufen gelassen. Nach Zulaufende wurde eine Stunde bei 60°C gehalten und nach Entnahme einer Probe 148 $cm^3$ = 134,5 g bzw. 89 $cm^3$ = 81 g Styrol zugegeben und zwischen 50 und 60°C auspolymerisiert.

Nach 60 Minuten wurde auf 40°C abgekühlt, eine weitere Probe entnommen und die orangefarbene Polymerisationslösung mit 1-n-Isopropanollösung in Cyclohexan mit einer kalibrierten Spritze auf farblos titriert, um die bei Polymerisationsende noch vorhandene-Katalysatorreaktivität zu bestimmen. Die abgebrochene Lösung wurde dann durch Eingießen in 5 l Ethanol, das 0,5 Gew.% Di-t-butyl-p-kresol enthielt, gefällt. Nach mehrfachem Auskneten mit Alkohol wurden die Polymeren bei 60°C über Nacht im Vakuumtrockenschrank getrocknet.

Die folgenden Tabellen 5 und 6 geben die analytischen Meßwerte und die mechanischen Eigenschaften wieder.

Tabelle 5

Polymerisation von 3-Block Styrol-Butadien-Styrol-Copolymeren: Analytische Daten

| Beispiel Nr. | Katalysator aus Beispiel Nr. | Polares Lösungsmittel (PLM) (LM: Cyclohexan) | | | Starttemp. der Polym. | PA in % der Ausgangs PA 1) | VZ des Endproduktes | GPC-MGW's x $10^{-3}$ | | | | | | | | Bemerkung |
|---|---|---|---|---|---|---|---|---|---|---|---|---|---|---|---|---|
| | | | | | | | | PB-Block | | Endprodukt | | OsO4-Abbau Polystyrol | | | | |
| | | | | | | | | | | | | Gehalt-Gew.% | | MGW x $10^{-3}$ aus VZ | | |
| | | Name | Mol-Verhältnis PLM:PA | Konzent. im LM m Mol/l | | | | soll | gef. 2) | soll | gef. 2) | soll | gef. | soll 3) | gef. | |
| 32 | 1 | THF | 20 | 172 | 20° | -- | 33,4 | 10 | 14 | 20 | 22 | 50 | 53,1 | 5,5 | 8,5 | Prod. zäh + elastisch |
| 33 | 3a | THF | 1,54 | 15,3 | 65° | -- | 96,6 | 45 | 65 | 60 | 80 | 27 | 26,2 | 9 | 11,5 | Prod. zäh + elastisch |
| 34 | 5a | THF | 1,5 | 8,3 | 65° | -- | 95,7 | 45 | 55 | 60 | 80 | 27 | 28,8 | 12 | 13 | Prod. zäh + elastisch |
| 35 | 9 | THF | 6 | 28 | 40° | 97 | 93,4 | 45 | 67 | 60 | 90 | 27 | 26,1 | 12,2 | 11 | Prod. zäh + elastisch |
| 36 | 9a | THF | 1,5 | 6,7 | 40° | 95 | 107,5 | 45 | 70 | 60 | 95 | 27 | 26,9 | 12,8 | 11 | Prod. zäh + elastisch |
| 37 | 10 | DEE | 2 | 9,5 | 40° | 97 | 96,7 | 45 | 76 | 60 | 100 | 27 | 26,0 | 13,5 | 13 | Prod. zäh + elastisch |
| 38 | 10a | DEE | 0 | 0,0 | 75° | 96 | 123,1 | 45 | 70 | 60 | 95 | 27 | 24,8 | 12,8 | 12 | Prod. zäh + elastisch |
| 39 | 11 | THF | 2 | 9,4 | 40° | 97 | 98,8 | 45 | 70 | 60 | 95 | 27 | 26,0 | 12,8 | 12 | Prod. zäh + elastisch |
| 40 | 13 | DEE | 4 | 17,6 | 65° | 87,5 | 110,0 | 45 | 80 | 60 | 110 | 27 | 25,9 | 14,9 | 12 | Prod. zäh + elastisch |
| 41 | 13a | DEE | 0,09 | 0,41 | 74° | 90 | 130,5 | 45 | 80 | 60 | 100 | 27 | 27,2 | 13,5 | 17 | Prod. zäh + elastisch |
| 42 | 14a | DEE | 0,02 | 0,1 | 75,5° | 97 | 135,4 | 45 | 88 | 60 | 120 | 27 | 26,5 | 16,2 | 17 | Prod. zäh + elastisch |
| 43 | 15 | DEE | 4 | 17,6 | 74,5 | 97 | 134,4 | 45 | 82 | 60 | 110 | 27 | 28 | 14,9 | 14 | Prod. zäh + elastisch |
| 44 | 15a | DEE | 0,17 | 0,8 | 75 | 97,5 | 136,8 | 45 | 90 | 60 | 120 | 27 | 25,4 | 16,2 | 17 | Prod. zäh + elastisch |

1) Ermittelt nach Abschluß der Polymerisation durch Rücktitration mit Isopropanol - siehe Text.

2) Errechnet aus dem Verhältnis Initiator zu Monomer für Mw/Mn = 1

3) Das MGW der Polystyrolblöcke wurde für das durch GPC ermittelte MGW der Endprodukte der Polymerisation errechnet unter der Annahme, daß beide Kettenenden aus Polystyrolblöcken bestehen.

THF - Tetrahydrofuran
DEE - Diethylether

Tabelle 6
Mechanische Eigenschaften und Konfiguration

| Nr. | Zugfestigkeit bei 300 % Dehnung n/mm2 | Reißfestigkeit N/mm2 | Reißdehnung (%) | Butadienkonfiguration FTIR 2) % 1,4-trans | % 1,2- | % 1,4-cis |
|---|---|---|---|---|---|---|
| 38 | 2,28 | 11,2 | 1660 | 58,2 | 10 | 31,8 |
| 40 | 2,42 | 12,4 | 1340 | 32,5 | 46 | 21,5 |
| 41 | 2,25 | 11,6 | 1352 | 59 | 11 | 30 |
| Vergleich 1) | 1,95 | 9,62 | 577 | 57,4 | 12,2 | 30,4 |

1) Das SBS-3-Blockcopolymerisat, 27 Gew.% Styrol, 73 Gew.% Butadien und MGW: 68000, wurde mit Lithiumbutyl durch aufeinanderfolgende Polymerisation von Styrol - Butadien - Styrol in Cyclohexan bei 70°C nach bekannten Methoden hergestellt.

2) FTIR = Fourieranalyse der Infrarotspektren

Aus den Daten geht hervor, daß die mit erfindungsgemäßen Katalysatoren hergestellten Polymeren dem Vergleich in den mechanischen Eigenschaften überlegen sind.

Beispiel 45

Ein mit Magnetrührer, Wasserbad, Thermometer und Stickstoffspülung ausgerüsteter 500 $cm^3$ Kolben wurde mit 100 $cm^3$ gereinigtem Toluol beschickt, mit einer kalibrierten Spritze durch eine Gummimembrane mit der Katalysatorlösung von Beispiel 30 (Dinatriumkatalysator) auf schwachen Orangefarbton austitriert und sofort 0,77 $cm^3$ (0,5 mmol PA) der Katalysatorlösung zugegeben. Bei 50°C wurde auspolymerisiert, das Polystyrol durch Eingießen bei intensivem Rühren in Ethanol gefällt, auf der Nutsche mit Alkohol gewaschen und getrocknet. Durch GPC wurde ein MGW von 63000 (ber. 40000) bestimmt.

Beispiel 46

Herstellung eines durch Aninoendgruppen funktionalisierten Polybutadienöles

In der Apparatur gemäß Beispiel 32, die jedoch mit einem Kreuzbalkenmetallrührer für besonders gute Durchmischung und Anwendung eines hohen Drehmomentes ausgestattet war, wurden 3000 cm3 Cyclohexan und 50 $cm^3$ Butadien bei 65°C zugegeben, 16,5 mmol des Katalysators (33 mmol PA) aus Beispiel 26 zugegeben, und bei dieser Temperatur weitere 83 $cm^3$ (zusammen 83 g) Butadien so zugefahren, daß gerade kein Rückfluß auftritt. Nach Beendigung des Zulaufes wird zur Vervollständigung der Polymerisation noch 30 Minuten bei 60°C gehalten, dann auf 40°C abgekühlt und mit 40 mmol Propylendiaziridin funktionalisiert, wobei die Lösung gallertartig viskos wird. Nach 30 Minuten Rühren wird der Reaktorinhalt mit 2 l Ethanol gefällt. Das sich absetzende Polybutadienöl wird 3 mal mit Methanol ausgerührt, mit 0,2 g Di-t-butyl-p-kresol gemischt und in Vakuum bei 60°C getrocknet. Es entsteht ein gießbares Polybutadienöl vom MGW 6000 (GPC) und enger

MGW-Verteilung. Der Stickstoffgehalt nach Kjeldahl beträgt 0,85 % (Theorie: 0,93 %). Die FTIR-Analyse zeigt eine Konfiguration von 41,4 % 1,2-, 23,4 % 1,4-cis und 35,5 % 1,4-trans-Struktur für das eingebaute Polybutadien.

In 5 g des Öles wurden in einen Penicillinglas bei Zimmertemperatur mit einem Glasstab schnell 4 mmol Hexamethylendiisocyanat (0,6 g) eingerührt und auf Silikonpapier gegossen. Die Mischung erstarrt in Minuten zu einem farblosen, elastischen Gummi.

Beispiel 47

Herstellung eines durch OH-Gruppen funktionalisierten Polybutadienöls

Es wurde gearbeitet wie im Beispiel 46 beschrieben, jedoch wurde statt mit Propylenaziridin mit 40 mmol Ethylenoxid funktionalisiert. Dabei tritt eine noch stärkere Erhöhung der Viskosität als in Beispiel 46 auf, und die Masse nimmt eine aspikähnliche Konsistenz an. Nachdem man 1 Std. gerührt hat, um eine vollständige Durchmischung und Reaktion zu gewährleisten, wird durch Zugabe von einigen cm3 Methanol abgebrochen, wobei eine wasserdünne Lösung entstehen. Das eingebaute Polybutadien hat 39 % 1,2-, 24 % cis-1,4- und 37 % trans-1,4-Konfiguration. Das Polybutadienöl hat ein MGW(GPC) von 6200 und eine OH-Zahl von 16.

## Patentansprüche

1. Verwendung einer mit Alkalimetall umgesetzten Stilbenverbindung der allgemeinen Formel Ia, Ib oder Ic

$$R^1R^2R^3Ar^1\text{-}CH{=}CH\text{-}Ar^2R^4R^5R^6 \qquad (Ia)$$

$$R^1R^2R^3Ar^1\text{-}CH{=}CH\text{-}Ar^2R^4R^5R^6\text{-}CH{=}CH\text{-}Ar^3R^7R^8R^9 \qquad (Ib)$$

$$R^1R^2R^3Ar^1\text{-}CH{=}CH\text{-}Ar^2(CH_2)_nAr^3\text{-}CH{=}CH\text{-}Ar^4R^4R^5R^6 \qquad (Ic)$$

in der $Ar^1$ bis $Ar^4$ gleiche oder verschiedene aromatische oder quasi-aromatische Reste und n die Zahl 0 bis 20 bedeutet und in der entweder wenigstens einer der Reste $R^1$ bis $R^6$ bzw. $R^9$ einen in Kohlenwasserstoffen löslichkeitsvermittelnden Alkyl-, Alkoxy-, Dialkylamino- oder Diarylaminorest mit wenigstens 4 Kohlenstoffatomen im Alkylteil bedeutet oder, wenn in der Formel Ic keiner der Reste $R^1$ bis $R^6$ vorhanden ist, die Zahl n wenigstens 4 bedeutet, als Katalysator für die anionische Polymerisation.

2. Verfahren zur Herstellung von Verbindungen der allgemeinen Struktur Ib oder Ic nach Anspruch 1, dadurch gekennzeichnet, daß man

   a) ein Toluol oder Xylol bzw. eine toluol/xylolanaloge Verbindung $R^1R^2R^3ArCH_3$ bzw. $H_3CR^4R^5R^6ArCH_3$ metalliert,

   b) mit einem entsprechenden Dialdehyd OHC-Ar-CHO umsetzt, wobei entsprechend substituierte Metalldialkoholate des Typs IIb bzw. IIb′ gebildet werden,

$$ArCH(OM)CH_2ArCH_2CH(OM)Ar \qquad IIb$$

$$ArCH_2CH(OM)ArCH(OM)CH_2Ar \qquad IIb'$$

   und diese

   c) hydrolysiert/solvolysiert und dehydratisiert oder pyrolysiert,

   wobei die Herstellung von Verbindungen der Formel Ib, in der $R^1$, $R^2$, $R^3$, $R^7$, $R^8$ und/oder $R^9$ Alkyl oder Alkoxy bedeuten, ausgenommen ist.

3. Verfahren nach Anspruch 2, dadurch gekennzeichnet, daß man als Metallierungsmittel n-Alkyllithium verwendet.

4. Verfahren nach Anspruch 2, dadurch gekennzeichnet, daß man die Solvolyse mit Essigsäureanhydrid vornimmt.

5. Verbindung der allgemeinen Formel Ib oder Ic nach Anspruch 1.

## Claims

1. Use of an alkali-metalated stilbene compound of the general formula Ia, Ib or Ic

$$R^1R^2R^3Ar^1\text{-}CH{=}CH\text{-}Ar^2R^4R^5R^6 \qquad (Ia)$$

$$R^1R^2R^3Ar^1\text{-}CH{=}CH\text{-}Ar^2R^4R^5R^6\text{-}CH{=}CH\text{-}Ar^3R^7R^8R^9 \qquad (Ib)$$

$R^1R^2R^3Ar^1\text{-}CH=CH\text{-}Ar^2(CH_2)_nAr^3\text{-}CH=CH\text{-}Ar^4R^4R^5R^6$ (Ic)

where $Ar^1$ to $Ar^4$ are identical or different aromatic or quasi-aromatic radicals and n is from 0 to 20 and where either at least one of the radicals $R^1$ to $R^6$ or $R^9$ is hydrocarbon-solubilizing alkyl, alkoxy, dialkylamino or diarylamino of 4 or more carbon atoms in the alkyl moiety or, if formula Ic contains no radicals $R^1$ to $R^6$, n is not less than 4, as catalyst for anionic polymerization.

2. A process for preparing a compound of the general structure Ib or Ic as claimed in claim 1, which comprises
a) metalating a toluene or xylene or a toluene/xylene analog $R^1R^2R^3ArCH_3$ or $H_3CR^4R^5R^6ArCH_3$,
b ) reacting with an appropriate dialdehyde OHC-Ar-CHO to form a correspondingly substituted metal dialcoholate of type IIb or IIb′

$ArCH(OM)CH_2CH_2ArCH(OM)Ar$ IIb

$ArCH_2CH(OM)ArCH(OM)CH_2Ar$ IIb

and
c) hydrolyzing/solvolyzing and dehydrating or pyrolyzing this intermediate, but not a compound of the formula Ib where $R^1$, $R^2$, $R^3$, $R^7$, $R^8$ and/or $R^9$ are each alkyl or alkoxy.

3. A process as claimed in claim 2, wherein the metalating agent used is an n-alkyllithium.

4. A process as claimed in claim 2, wherein solvolysis is performed with acetic anhydride.

5. A compound of the formula Ib or Ic as claimed in claim 1.

## Revendications

1. Utilisation d'un dérivé du stilbène, ayant réagi avec un métal alcalin, de formule générale Ia, Ib ou Ic

$R^1R^2R^3Ar^1\text{-}CH=CH\text{-}Ar^2R^4R^5R^6$ (Ia)

$R^1R^2R^3Ar^1\text{-}CH=CH\text{-}Ar^2R^4R^5R^6\text{-}CH=CH\text{-}Ar^3R^7R^8R^9$ (Ib)

$R^1R^2R^3Ar^1\text{-}CH=CH\text{-}Ar^2(CH_2)_nAr^3\text{-}CH=CH\text{-}Ar^4R^4R^5R^6$ (Ic)

où les radicaux $Ar^1$ à $Ar^4$ sont des radicaux aromatiques ou quasi-aromatiques identiques ou différents, et n représente un nombre de 0 à 20, et où au moins l'un des radicaux $R^1$ à $R^6$ ou $R^9$ représente un radical alkyle, alcoxy, dialkylamino ou diarylamino rendant soluble dans les hydrocarbures, ayant au moins 4 atomes de carbone dans le fragment alkyle, ou encore, quand la formule Ic ne contient aucun des radicaux $R^1$ à $R^6$, le nombre n vaut au moins 4, en tant que catalyseur pour la polymérisation anionique.

2. Procédé pour préparer des composés ayant la structure générale Ib ou Ic selon la revendication 1, caractérisé en ce que :
a) on métalle un toluène ou un xylène ou un composé analogue au toluène/xylène $R^1R^2R^3ArCH_3$ ou $H_3CR^4R^5R^6\text{-}ArCH_3$,
b) on le fait réagir avec un dialdéhyde correspondant OHC-Ar-CHO, ce à l'occasion de quoi il se forme des dialkylates métalliques du type IIb ou IIb′, substitués en conséquence

$ArCH(OM)CH_2ArCH_2CH(OH)Ar$ IIb

$ArCH_2CH(OM)ArCH(OM)CH_2Ar$ IIb′

et
c) on les hydrolyse/solvolyse et on les déshydrate ou on les pyrolyse,
et ce à l'exclusion de la préparation des composés de formule IB dans laquelle $R^1$ , $R^2$ , $R^3$ , $R^7$ , $R^8$ , et/ou $R^9$ représentent des radicaux alkyle ou alcoxy.

3. Procédé selon la revendication 2, caractérisé en ce qu'on utilise en tant qu'agent de métallation un n-alkyllithium.

4. Procédé selon la revendication 2, caractérisé en ce qu'on procède à la solvolyse à l'aide d'anhydride acétique.

5. Composé de formule générale Ib ou Ic selon la revendication 1.